# EUROPÄISCHE PATENTSCHRIFT

(11) **EP 4 637 665 B1**
(45) Veröffentlichungstag und Bekanntmachung des Hinweises auf die Patenterteilung: **04.11.2026**
(21) Anmeldenummer: 23834034.3
(22) Anmeldetag: 18.12.2023
(51) Int. Cl.: A61F 13/514, A61F 13/496

(54) **ABSORBIERENDER INKONTINENZARTIKEL IN HÖSCHENFORM**
ABSORBENT INCONTINENCE ARTICLE IN THE FORM OF BRIEFS
ARTICLE ABSORBANT POUR INCONTINENCE SOUS FORME DE SOUS-VÊTEMENT

(30) Priorität: 22.12.2022 DE 102022134647
(43) Veröffentlichungstag der Anmeldung: 29.10.2025
(73) Patentinhaber: Paul Hartmann AG, 89522 Heidenheim (DE)
(72) Erfinder: CIFRIC, Nedzad, 89522 Heidenheim (DE); KAUTZSCH, Florian, 89522 Heidenheim (DE); EILERS, Jörg, 89522 Heidenheim (DE); BUCH, Tamara, 89522 Heidenheim (DE)
(74) Vertreter: Paul Hartmann AG Patents & Licensing
(86) Internationale Anmeldenummer: PCT/EP2023/086252
(87) Internationale Veröffentlichungsnummer: WO 2024/133005

(56) Entgegenhaltungen:
- JP-A- 2018 518 240
- US-A1- 2014 163 509
- US-A1- 2017 007 470

## Beschreibung

Die vorliegende Erfindung betrifft einen absorbierenden Inkontinenzartikel in Höschenform für die Aufnahme von Körperausscheidungen umfassend einen vorderen Bauchabschnitt und einen hinteren Rückenabschnitt, die in einer Längsrichtung über eine Distanz D voneinander beabstandet sind und die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, und einen Schrittabschnitt, der sich in der Längsrichtung zwischen Bauchabschnitt und Rückenabschnitt erstreckt und an den Bauchabschnitt in einem vorderen Überlappungsbereich und an den Rückenabschnitt in einem hinteren Überlappungsbereich unlösbar angefügt ist, wobei der Schrittabschnitt einen Absorptionskörper und ein flüssigkeitsundurchlässiges Backsheet-Material aufweist und wobei der Schrittabschnitt, der Bauchabschnitt und der Rückenabschnitt Beinöffnungen des Inkontinenzartikels begrenzen, wobei auf einer körperabgewandten Oberseite des Backsheet-Materials eine in Längsrichtung entlang der Distanz D und sich bis in den vorderen und hinteren Überlappungsbereich erstreckende Vlieslage vorgesehen ist, wobei die Vlieslage in Längsrichtung vor einem vorderen Ende des Backsheet-Materials und/oder vor einem hinteren Ende des Backsheet-Materials endet.

Es handelt sich hierbei um einen Inkontinenzartikel in Höschenform mit einem modularen, dreikomponentigen Aufbau mit den Hauptkomponenten Bauchabschnitt, Rückenabschnitt und Schrittabschnitt. Ein derartiger Inkontinenzartikel ist beispielsweise bekannt aus DE 10 2007 055 628 A1.

Die Vlieslage unterstützt dabei den textilen Charakter des Inkontinenzartikels und damit ein positives Tragefühl.

Die Ausstattung der körperabgewandten Oberseite des Backsheet-Materials mit einer Vlieslage und dabei in einer gegenüber dem Backsheet-Material verkürzten Erstreckung in Längsrichtung zeigen beispielsweise die Dokumente EP2849696B1, EP2105115B1 und EP3454808B1.

Weiterer Stand der Technik ist aus US2017/007470 bekannt.

Es wurde erkannt, dass im Anwendungszustand des Inkontinenzartikels auf den Schrittbereich Kräfte ausgeübt werden. Bei Anordnung von Lagen übereinander kann dies unvorteilhaft zu einem Abschälen der Lagen voneinander führen. Durch eine Delaminierung hervorgerufene freistehende Ränder der einzelnen Lagen können unvorteilhaft unerwünschte Umfaltungen mit sich bringen, die Hautirritationen hervorrufen als auch Schwachstellen im Inkontinenzartikel erzeugen können.

Der vorliegenden Erfindung liegt die Aufgabe zugrunde, bei einem Inkontinenzartikel der gattungsgemäßen Art den Lagenverbund aus Vlieslage und Backsheet-Material im Hinblick auf die in der Anwendung auftretende Belastungssituationen zu verbessern und einen hohen Tragekomfort zu gewährleisten.

Diese Aufgabe wird erfindungsgemäß gelöst durch einen Inkontinenzartikel mit den Merkmalen des Anspruchs 1.

Dabei ist die Vlieslage mittels eines ersten und eines zweiten in Längsrichtung erstreckten randständigen Längskleberstreifens und mittels einer Vielzahl bezüglich der randständigen Längskleberstreifen in Querrichtung zwischen den randständigen Längskleberstreifen innenliegenden und in Längsrichtung erstreckten, parallel zueinander und voneinander durch kleberfreie Längsstreifen beabstandeten ersten Längskleberstreifen mit dem Backsheet-Material verbunden, wobei in Querrichtung die randständigen Längskleberstreifen eine Breite B14, die ersten Längskleberstreifen eine Breite B12 und die kleberfreien Längsstreifen eine Breite B10 aufweisen.

"Randständig" bedeutet aber nicht, dass sich der randständige Längskleberstreifen mit seinem äußeren Längsrand zwingend bis zum jeweiligen geometrischen Rand der Vlieslage und/oder des Backsheet-Materials erstrecken muss. Vielmehr ist es bevorzugt, dass der randständige Längskleberstreifen einen gewissen Abstand in einer Größenordnung eines oder weniger Millimeter vom geometrischen Rand der Vlieslage und/oder des Backsheet-Materials einhält, um zu vermeiden, dass Kleber aus den Randbereichen von Vlieslage und/oder Backsheet-Material austritt und eine Verunreinigung der Herstellungsvorrichtung verursacht wird oder auch eine Verunreinigung im fertigen Produkt sichtbar wird. Das analoge Verständnis wird auch für "randständig" im Zusammenhang mit randständigen Querkleberstreifen zugrunde gelegt.

Es wurde erkannt, dass bei Verwendung einer Vielzahl von in Längsrichtung des Inkontinenzartikels erstreckter Längskleberstreifen die im Anwendungszustand des Inkontinenzartikels und dabei in Querrichtung des Schrittbereichs typischerweise auftretenden Kräfte, wie Scherkräfte und Zusammenpressen gut aufgenommen werden können. Der Kräfteeintrag in den Randbereichen des Schrittabschnitts kann zum einen über die Längserstreckung von randständigen Kleberstreifen und weitergehend über die ersten Längskleberstreifen, die alternierend mit kleberfreien Längsstreifen angeordnet sind, abgeschwächt werden; zum anderen können die Kräfte über die Orientierung in Längsrichtung in Richtung Bauchabschnitt und/oder Rückenabschnitt, insbesondere in den Überlappungsbereich von Schrittabschnitt mit Bauchabschnitt und/oder mit Rückenabschnitt geleitet werden. Dies ist vorteilhaft für eine Verbundverfestigung der Lagen.

Weiter vorteilhaft kann mit der alternierenden Anordnung von Längskleberstreifen und kleberfreien Längsstreifen im Bereich der Überlappung von Vlieslage mit dem Backsheet-Material eine vollflächige Versteifung vermieden werden. Vielmehr wird mittels der kleberfreien Längsstreifen eine Flexibilität in Querrichtung erreicht, was im Anwendungszustand des Inkontinenzartikels beim Zusammenpressen des Schrittabschnitts in Querrichtung vorteilhaft ist. Es kann damit eine Art "Schiffchenbildung", also eine Aufeinanderfaltung des Schrittabschnitts entlang der Längsrichtung unterstützt werden.

Des Weiteren können mit der alternierenden Anordnung von Längskleberstreifen und kleberfreien Längsstreifen zwischen Vlieslage und dem davon innenliegendem Backsheet-Material haptisch und/oder visuell unterscheidbare Bereiche auf der Außenseite des Inkontinenzartikel erreicht werden, die neben dem textilen Charakter auch das Verhalten der Vlieslage in Zusammenschau mit über dem Inkontinenzartikel getragenen Kleidungsstücken vorteilhaft beeinflussen können. Mit den in Längsrichtung verlaufenden und in Querrichtung beabstandeten kleberhaltigen und kleberfreien Bereichen wird ein Reibungskrafteintrag und damit Haftung in Querrichtung eher erreicht als in Längsrichtung. Ein geringerer Reibungseintrag in Längsrichtung ermöglicht ein besseres Gleiten des Inkontinenzartikels gegenüber dem Kleidungsstück in Längsrichtung, hingegen ein höherer Reibungseintrag und Haftung in Querrichtung den Verbleib des Inkontinenzartikels auf der Kleidung, was insbesondere im Schrittbereich und über dessen Breite wünschenswert ist, unterstützt.

Ferner kann mit einer Anordnung von einer Vielzahl an in Längsrichtung erstreckter Längskleberstreifen alternierend zu kleberfreien Längsstreifen für die Bindung zwischen der Vlieslage und dem Backsheet-Material die Menge der erforderlichen Klebermaterialien reduziert werden verglichen mit einem vollflächigen Kleberauftrag, was der Kosteneinsparung zuträglich ist, ohne die für den Tragezustand notwendige Verbundfestigkeit zu minimieren.

Des Weiteren wird mit der erfindungsgemäßen Kleberstreifen-Anordnung eine Erhöhung der Atmungsaktivität im Vergleich zu einer vollflächigen Beleimung vorteilhaft unterstützt. Mit der Anordnung von Längskleberstreifen und kleberfreien Längsstreifen in Längsrichtung verbleiben im Bereich der kleberfreien Längsstreifen zwischen Vlieslage und Backsheet-Material sich in Längsrichtung erstreckende kanal- bzw. tunnelartige Bereiche, die die Atmungsaktivität weiter vorteilhaft unterstützen können durch die Möglichkeit eines Transports des sich im Miktionspunktes, insbesondere im Schrittbereich ansammelnden Wasserdampfes vom Miktionspunkt weg in Längsrichtung in Richtung Bauch und Rücken. Wohingegen eine Anordnung von Kleberstreifen und kleberfreien Streifen in Querrichtung diesen Transfer des Wasserdampfes weniger unterstützen könnte und die Feuchtigkeit vielmehr im Bereich des Miktionspunktes bzw. in Querrichtung um den Miktionspunkt, insbesondere im Schrittbereich verbleiben würde.

Zudem kann bei der gattungsgemäßen Art des Inkontinenzartikels mit Modulaufbau bei der Herstellung des Schrittabschnitts und dabei bei der Anfügung der Vlieslage an das Backsheet-Material der Kleberauftrag für die Längskleberstreifen vorteilhaft in Maschinenrichtung geführt werden.

Die Einbringung der Längskleberstreifen kann mittels Schlitzdüsen-Auftragsvorrichtungen durchgeführt werden. Auch unter Verwendung einer Schablone aus Blech mit Aussparungen ist die Aufbringung von Kleber als Längskleberstreifen alternierend zu kleberfreien Längsstreifen möglich. Weiter insbesondere erfolgt der Auftrag an Kleber dabei auf eine die spätere Vlieslage bereitstellende Materialbahn, damit Kleber tatsächlich nur in einer Überlappungszone von Vlieslage mit Backsheet-Material vorhanden ist.

Bei einem gattungsgemäßen dreikomponentigen Modulaufbau des Inkontinenzartikels ergibt sich im Bereich des Übergangs zwischen Schrittabschnitt und Bauchabschnitt und/oder im Bereich des Übergangs zwischen Schrittabschnitt und Rückenabschnitt im Vergleich zu Inkontinenzartikeln mit einem vollflächigen Außenchassismaterial ein zusammengesetzter Verlauf der die Beinöffnung begrenzenden Ränder. Dies birgt die Gefahr, dass bei Anwendung des Inkontinenzartikels im Bereich der Übergänge Kraftspitzen gebildet werden, die eben zu einem Einreißen der Chassismaterialien führen können und damit auch die Anfügung des Schrittabschnitts an den Bauchabschnitt und/oder die Anfügung des Schrittabschnitts an den Rückenabschnitt beeinträchtigen kann.

Vorzugsweise erstrecken sich die randständigen Längskleberstreifen in Längsrichtung derart, dass die randständigen Längskleberstreifen in einem vorderen Übergangsbereich des Schrittabschnitts, der sich in Längsrichtung vor und hinter einem schrittzugewandtem Rand des Bauchabschnitts erstreckt, vorhanden sind und dass die randständigen Längskleberstreifen in einem hinteren Übergangsbereich des Schrittabschnitts, der sich in Längsrichtung vor und hinter einem schrittzugewandten Rand des Rückenabschnitts erstreckt, vorhanden sind. Damit kann zu einer Verstärkung der beiden Materiallagen Vlieslage und Backsheet-Material beigetragen werden und damit der Gefahr des Einreißens im Bereich des Übergangs entgegengewirkt werden.

Weiter vorzugsweise erstrecken sich die ersten Längskleberstreifen in Längsrichtung in dem Maße, dass erste Längskleberstreifen in dem vorderen Übergangsbereich des Schrittabschnitts und/oder in dem hinteren Übergangsbereich des Schrittabschnitts vorgesehen sind.

Beidseitige und die Beinöffnungen begrenzende Randbereiche des Schrittabschnitts können in Längsrichtung ausschließlich gerade oder zumindest entlang eines Teils der beidseitigen Randbereiche des Schrittabschnitts bogenförmig konturiert verlaufen.

Bei einer bevorzugten Ausführung im Falle ausschließlich gerader Randbereiche des Schrittabschnitts erstrecken die randständigen Längskleberstreifen sich zumindest über die Distanz D kontinuierlich und weiter insbesondere erstrecken sich einige der ersten Längskleberstreifen, weiter insbesondere alle ersten Längskleberstreifen zumindest über die Distanz D kontinuierlich.

Im Falle bogenförmiger Konturierung der beidseitigen Randbereiche des Schrittabschnitts können die randständigen Längskleberstreifen und insbesondere erste Längskleberstreifen, die den randständigen Längskleberstreifen unmittelbar benachbart angeordnet sind, im Bereich der Konturierung in Längsrichtung unterbrochen sein.

Vorzugsweise sind die Längsklebersteifen und die kleberfreien Längsstreifen über die gesamte Erstreckung in Querrichtung der Vlieslage angeordnet.

Vorzugsweise weisen außerhalb eines ersten und zweiten Längsrands des Absorptionskörpers das Backsheet-Material und die Vlieslage einen sich in Querrichtung erstreckenden ersten und zweiten Überhang auf, wobei der erste und zweite randständige Längskleberstreifen innerhalb des jeweiligen ersten und zweiten Überhangs und in einem Abstand zum jeweiligen Längsrand des Absorptionskörpers angeordnet sind.

In einer bevorzugten Ausführung weist der Schrittabschnitt im Bereich von Randbereichen des Schrittabschnitts, vorzugsweise innerhalb des ersten und zweiten Überhangs, sich in Längsrichtung erstreckende Schrittelastifizierungsmittel auf.

Die Fixierung der Schrittelastifizierungsmittel kann vorzugsweise in randständigen Bereichen des Backsheet-Materials erfolgen. Die Längskleberstreifen, vor allem die randständigen Längskleberstreifen, können dabei als eine partielle Verstärkung in den randständigen Bereichen des Backsheet-Materials gegenüber den durch die elastische Wirkung der Schrittelastifizierungsmittel in den Verbund mit dem Backsheet-Material eingebrachten Kräfte positiv zuträglich sein.

Vorzugsweise ist vorgesehen, dass ein Verhältnis der Breite B12 von ersten Längskleberstreifen zur Breite B10 von unmittelbar benachbarten kleberfreien Längsstreifen, berechnet als B12/B10, insbesondere 0,2 - 3,0, insbesondere 0,5 - 2,5, weiter insbesondere 0,7- 2,5, weiter insbesondere 0,9 - 2,3, weiter insbesondere 1,0 - 2,0 beträgt. Damit wird in vorteilhafter Weise eine fein und gleichmäßig verteilte Abfolge von Kleberstreifen und kleberfreien Streifen bereitgestellt, was eine gleichmäßige Fügebereichsverteilung und damit verbunden eine Fügestabilität zwischen den Lagen bei gleichmäßiger Verteilung einer möglichen Versteifung durch Kleberstreifen bereitstellt.

Die Breite B12 der ersten Längskleberstreifen in Querrichtung beträgt vorzugsweise wenigstens 1 mm bis höchstens 10 mm, insbesondere bis höchstens 8 mm, weiter insbesondere bis höchstens 6 mm, weiter insbesondere bis höchstens 4 mm.

Die Breite B10 der kleberfreien Längsstreifen in Querrichtung beträgt vorzugsweise wenigstens 1 mm bis höchstens 12 mm, insbesondere bis höchstens 10 mm, insbesondere bis höchstens 8 mm, weiter insbesondere bis höchstens 6 mm, weiter insbesondere bis höchstens 4 mm.

Vorzugsweise ist die Breite B12 der ersten Längskleberstreifen jeweils gleich groß und/oder ist die Breite B10 der kleberfreien Längsstreifen jeweils gleich groß.

Alternativ kann auch vorgesehen sein, dass die Breite B12 eines außen liegenden ersten Längskleberstreifens größer ist als die Breite B12 des in Querrichtung unmittelbar benachbarten davon innen liegenden ersten Längskleberstreifens. Insbesondere kann vorgesehen sein, dass die Breite B12 eines äußersten ersten Längskleberstreifens größer ist als die Breite B12 der in Querrichtung davon innen liegenden ersten Längskleberstreifen. Insbesondere kann vorgesehen sein, dass die Breite B12 eines äußersten ersten Längskleberstreifen größer ist als die Breite B12 eines innersten ersten Längskleberstreifens.

Vorzugsweise kann die Gestaltung der Breite B12 der ersten Längskleberstreifen und der Breite B10 der kleberfreien Längsstreifen dergestalt sein, dass die Breite B12 von äußeren ersten Längskleberstreifen größer ist als die Breite B12 von inneren ersten Längskleberstreifen und dass die Breite B10 von äußeren kleberfreien Längsstreifen kleiner ist als die Breite von inneren kleberfreien Längsstreifen.

Mit dieser Differenzierung der Breite B12 der ersten Längskleberstreifen innerhalb der Vielzahl an ersten Längskleberstreifen kann zum einen der Verbundfestigkeit von Vlieslage mit Backsheet-Material entsprechend dem höheren Kräfteeintrag in den äußeren Randbereichen als auch dem Komfort mit Flexibilität, Atmungsaktivität in den inneren mittleren Bereichen positiv Rechnung getragen werden.

Alternativ kann insbesondere dennoch auch vorgesehen sein, dass zumindest ein innerster, insbesondere zwei innerste erste Längskleberstreifen eine Breite B12 aufweisen, die größer ist als die Breite B12 der davon außen liegenden ersten Längskleberstreifen. Somit kann vorteilhaft im Bereich der Längsmittelachse des Inkontinenzartikels, oftmals auch ein Bereich der Ansammlung von Kraftspitzen, die Verbundfestigkeit weiter unterstützt werden.

Mit "äußerem" und "innerem" ersten Längskleberstreifen wird die relative Anordnung der ersten Längskleberstreifen zueinander in Bezug zur Querrichtung bezeichnet, wobei ein äußerer erster Längskleberstreifen im Vergleich zu einem inneren ersten Längskleberstreifen in Bezug zur Querrichtung weiter entfernt von einer Längsmittelachse des Inkontinenzartikels angeordnet ist. Unter dem "äußersten ersten Längskleberstreifen" wird der dem randständigen Längskleberstreifen unmittelbar benachbart angeordnete erste Längskleberstreifen und damit der Längsmittelachse am weitest entfernt gelegene erste Längskleberstreifen verstanden. Unter dem "innersten ersten Längskleberstreifen" wird der der Längsmittelachse am nächsten gelegene, unmittelbar benachbart angeordnete oder auf der Längsmittelachse angeordnete erste Längskleberstreifen verstanden.

Bei der Ausführung der ersten Längskleberstreifen und der randständigen Längskleberstreifen kann insbesondere vorgesehen sein, dass die randständigen Längskleberstreifen eine Breite B14 aufweisen, die zumindest so groß ist wie die Breite B12 der bezüglich der randständigen Längskleberstreifen innen liegenden ersten Längskleberstreifen.

Es kann dabei insbesondere vorgesehen sein, dass die Breite B14 der randständigen Längskleberstreifen der Breite B12 zumindest der zu den randständigen Längskleberstreifen unmittelbar benachbart angeordneten ersten Längskleberstreifen, insbesondere der Breite B12 von allen ersten Längskleberstreifen entspricht.

In einer bevorzugten Ausführung ist die Breite B14 der randständigen Längskleberstreifen größer als die Breite B12 der ersten Längskleberstreifen, insbesondere größer als die Breite B12 der zu den randständigen Längskleberstreifen unmittelbar benachbart angeordneten ersten Längskleberstreifen, weiter insbesondere größer als die Breite B12 von allen ersten Längskleberstreifen.

Insbesondere beträgt die Breite B14 der randständigen Längskleberstreifen dabei wenigstens das 1,5-fache, weiter insbesondere wenigstens das 2-fache, und weiter insbesondere höchstens das 20-fache, insbesondere höchstens das 15-fache, insbesondere höchstens das 10-fache, insbesondere höchstens das 8-fache, weiter insbesondere höchstens das 5-fache, insbesondere höchstens das 4-fache der Breite B12 der ersten Längskleberstreifen.

Eine im Vergleich zu den ersten Längskleberstreifen größeren Breite des ersten und zweiten randständigen Längsrandstreifens wird vorteilhaft gesehen, um ein Abschälen der Vlieslage vom Backsheet-Material unter Einfluss von seitlichen Krafteinwirkungen im Schrittbereich im Tragezustand des Inkontinenzartikels zu minimieren. Insbesondere in Bereichen des Übergangs von Schrittabschnitt mit dem Bauchabschnitt und/oder von Schrittabschnitt mit dem Rückenabschnitt ist eine partielle Verstärkung durch eine größere Breite der randständigen Längskleberstreifen vorteilhaft.

Alternativ kann insbesondere vorgesehen sein, dass die Breite B12 eines innersten ersten oder zwei innersten ersten Längskleberstreifen größer ist als die Breite B12 der übrigen ersten Längskleberstreifen und dabei die Breite B12 nicht größer ist als die Breite B14 der randständigen Längskleberstreifen.

Die Breite B14 der randständigen Längskleberstreifen beträgt vorzugsweise wenigstens 3 mm bis höchstens 20 mm, insbesondere bis höchstens 15 mm, weiter insbesondere bis höchstens 12 mm, weiter insbesondere bis höchstens 10 mm, weiter insbesondere bis höchstens 8 mm, weiter insbesondere bis höchstens 6 mm.

Mit der Vlieslage in Querrichtung eine maximale Breite B2 aufweisend, beträgt ein Verhältnis der Breite B12 der ersten Längskleberstreifen zur Breite B2, berechnet als B12/B2, vorzugsweise höchstens 0,10, vorzugsweise höchstens 0,08, weiter vorzugsweise höchstens 0,06, weiter vorzugsweise höchstens 0,04, weiter vorzugsweise höchstens 0,02. Mit Einsatz von ersten Längskleberstreifen von geringer Breite B12 kann vorteilhaft eine flächenhafte Versteifung reduziert werden.

Wenngleich den randständigen Längskleberstreifen eine Funktionalität der Verstärkung des Randbereichs zukommt, ist eine Abwägung von deren Breite B14 in Bezug auf die maximale Breite der Vlieslage vorzunehmen, um die dem Tragekomfort positiv beisteuernde Eigenschaften wie Atmungsaktivität und Flexibilität beibehalten zu können.

Die Vlieslage weist in Querrichtung eine maximale Breite B2 auf, wobei ein Verhältnis der Breite B14 der randständigen Längskleberstreifen zur Breite B2, berechnet als B14/B2, vorzugsweise 0,01 - 0,15, vorzugsweise 0,01 - 0,10, vorzugsweise 0,01 - 0,08, weiter vorzugsweise 0,01 - 0,06, weiter vorzugsweise 0,01 - 0,05, weiter vorzugsweise 0,01 - 0,04, weiter vorzugsweise 0,01 - 0,03 beträgt.

Die Vlieslage weist in Längsrichtung eine Länge L1 auf und das Backsheet-Material weist in Längsrichtung eine Länge L2 auf, wobei ein Verhältnis der Länge L1 zu Länge L2, berechnet als L1/L2, vorzugsweise mindestens 0,5, und weiter vorzugsweise höchstens 0,9, weiter vorzugsweise höchstens 0,8, weiter vorzugsweise höchstens 0,7 beträgt.

Eine Verkürzung der Vlieslage in Längsrichtung in Bezug zur Erstreckung des Backsheet-Materials in Längsrichtung trägt neben der Berücksichtigung des Kostenfaktors von Rohstoffmaterialien auch positiv dazu bei, dass in Längsendbereichen durch Vermeidung von kongruenten Längsenden aller Materiallagen abrupte Materiallagenstufen in den Endbereichen vermieden werden, sondern in Profilhöhe flacher auslaufende Endbereiche ein besser fließender Übergang zu anderen Komponenten des Inkontinenzartikels, wie insbesondere Bauchabschnitt und/oder Rückenabschnitt ermöglicht wird.

Vorzugsweise endet die Vlieslage in Längsrichtung vor einem vorderen Ende des Absorptionskörpers und/oder vor einem hinteren Ende des Absorptionskörpers.

Vorzugsweise weisen die ersten Längskleberstreifen und/oder die randständigen Längskleberstreifen ein Flächengewicht an Kleber von wenigstens 0,5 g/m², weiter insbesondere wenigstens 0,7 g/m², weiter insbesondere wenigstens 1 g/m², weiter insbesondere wenigstens 1,2 g/m², und insbesondere höchstens 10 g/m², weiter insbesondere höchstens 8 g/m², weiter insbesondere höchstens 6 g/m², weiter insbesondere höchstens 4 g/m² auf.

Es kann sich vorzugsweise vorteilhaft erweisen, wenn die randständigen Längskleberstreifen ein höheres Flächengewicht an Kleber aufweisen als die ersten Längskleberstreifen. Insbesondere vorteilhaft weisen die randständigen Längskleberstreifen ein Flächengewicht an Kleber auf, das mindestens das 2-fache, insbesondere mindestens das 3-fache, weiter insbesondere mindestens das 4-fache, weiter insbesondere mindestens das 5-fache und weiter insbesondere höchstens das 10-fache des Flächengewichts an Kleber der ersten Längskleberstreifen beträgt.

Mit einem höheren Flächengewicht an Kleber der randständigen Längskleberstreifen kann vorteilhaft eine bessere Haftung zwischen dem Backsheet-Material und der Vlieslage in Randbereichen erreicht werden und damit positiv gegen Probleme des Abschälens zwischen den beiden Lagen wirken.

In einer vorteilhaften Ausführung kann vorgesehen sein, dass die randständigen Längskleberstreifen ein Flächengewicht an Kleber aufweisen, das höher ist als das Flächengewicht des Klebers der ersten Längskleberstreifen, und wobei die Breite B14 der randständigen Längskleberstreifen mit der Breite B12 der ersten Längskleberstreifen übereinstimmt.

In einer anderen vorteilhaften Ausführung kann vorgesehen sein, dass die randständigen Längskleberstreifen ein Flächengewicht an Kleber aufweisen, das höher ist als das Flächengewicht des Klebers der ersten Längskleberstreifen, und wobei die Breite B14 der randständigen Längskleberstreifen größer ist als die Breite B12 der ersten Längskleberstreifen.

In einer alternativen Ausführung kann vorgesehen sein, dass die randständigen Längskleberstreifen ein Flächengewicht an Kleber aufweisen, das höher ist als das Flächengewicht des Klebers der ersten Längskleberstreifen, und wobei die Breite B14 der randständigen Längskleberstreifen geringer ist als die Breite B12 der ersten Längskleberstreifen.

Die Vlieslage überfängt das Backsheet-Material unter Bildung einer Überlappungszone, wobei die Überlappungszone eine Fläche A1 aufweist, und wobei vorzugsweise die ersten Längskleberstreifen und die randständigen Längskleberstreifen in Summe einen Bedeckungsgrad, berechnet als Gesamtfläche der Längskleberstreifen bezogen auf die Fläche A1 der Überlappungszone, von mindestens 10%, weiter insbesondere mindestens 12%, weiter insbesondere mindestens 14%, weiter insbesondere mindestens 16 %, und weiter insbesondere höchstens 80%, weiter insbesondere höchstens 75%, weiter insbesondere höchstens 70%, weiter insbesondere höchstens 65% aufweisen.

Bevorzugt ist die Ausführung, wenn die Vlieslage das Backsheet-Material in Querrichtung vollständig überfängt. Damit kann der textile Charakter, der Tragekomfort und auch die Verbundfestigkeit vorteilhaft unterstützt werden.

Alternativ kann vorgesehen sein, dass die Vlieslage in Querrichtung eine im Vergleich zum Backsheet-Material geringere Erstreckung in Querrichtung aufweist.

Der Schrittabschnitt ist an den Bauchabschnitt und den Rückenabschnitt unlösbar angefügt. Für die unlösbare Fixierung kann eine vollflächige Fügung, insbesondere eine vollflächige Verklebung, oder eine nicht vollflächige Fügung, insbesondere eine nicht vollflächige Verklebung gewählt werden.

Der Schrittabschnitt ist im vorderen und hinteren Überlappungsbereich vorzugsweise mit Orientierung der körperabgewandten Oberseite des Backsheet-Material an einer körperzugewandten Oberseite des Bauchabschnitts und des Rückenabschnitts angefügt.

In einer bevorzugten Ausführung ist der Schrittabschnitt an den Bauchabschnitt mittels einer Vielzahl von im vorderen Überlappungsbereich vorgesehener in Querrichtung erstreckter parallel zueinander und voneinander durch kleberfreie Querstreifen beabstandeter ersten Querkleberstreifen, die bezüglich optionaler randständiger Querkleberstreifen innen liegen, verbunden und/oder der Schrittabschnitt ist an den Rückenabschnitt mittels einer Vielzahl von im hinteren Überlappungsbereich vorgesehener in Querrichtung erstreckter parallel zueinander und voneinander durch kleberfreie Querstreifen beabstandeter ersten Querkleberstreifen, die bezüglich optionaler randständiger Querkleberstreifen innen liegen, verbunden.

Insbesondere sind im vorderen Überlappungsbereich und/oder im hinteren Überlappungsbereich in Querrichtung erstreckte randständige Querkleberstreifen vorgesehen, die in Längsrichtung eine Breite B18 aufweisen, wobei die Breite B18 vorzugsweise größer ist als eine Breite B16 der ersten Querkleberstreifen.

Insbesondere bevorzugt sind im vorderen Überlappungsbereich und/oder im hinteren Überlappungsbereich ein in Querrichtung erstreckter schrittzugewandter erster randständiger Querkleberstreifen und ein in Querrichtung erstreckter hüftöffnungszugewandter zweiter randständiger Querkleberstreifen und in Längsrichtung zwischen diesen eine Vielzahl von innenliegenden ersten Querkleberstreifen vorgesehen, wobei eine Breite B18 der randständigen Querkleberstreifen größer ist als eine Breite B16 der bezüglich der randständigen Querkleberstreifen innenliegenden ersten Querkleberstreifen, und insbesondere die Breite B18 dabei wenigstens das 4-fache, insbesondere wenigstens das 5-fache, und weiter insbesondere höchstens das 8-fache, insbesondere höchstens das 7-fache der Breite B16 der innenliegenden zweiten Querkleberstreifen beträgt.

Insbesondere bevorzugt überlappen in einem schrittzugewandten Randbereich des vorderen Überlappungsbereichs und/oder in einem schrittzugewandten Randbereich des hinteren Überlappungsbereichs zumindest der erste und zweite randständige Längskleberstreifen und weiter insbesondere einige der ersten Längskleberstreifen, weiter insbesondere alle ersten Längskleberstreifen mit einem schrittzugewandten ersten randständigen Querkleberstreifen.

Mit dieser Überlappung von randständigen Längskleberstreifen und von randständigen Querkleberstreifen ergeben sich bei einem gattungsgemäßen dreikomponentigen Modulaufbau des Inkontinenzartikels und den damit einhergehenden möglichen Schwachstellen in den Übergangsbereichen im Verlauf der die Beinöffnung begrenzenden Ränder durch den Eintrag von randständigem Klebermaterial vorteilhaft verstärkte Bereiche.

Der sich ergebende gitterförmige Kleberauftrag aus der alternierenden Abfolge von Längskleberstreifen und kleberfreien Längsstreifen auf der dem Backsheet-Material zugewandten Oberseite der Vlieslage und den Querkleberstreifen als Fügebereichskleber zwischen dem Bauch- bzw. Rückenabschnitt und der dem Backsheet-Material abgewandten Oberseite des Vlieslage ergibt in den schrittzugewandten Randbereichen des vorderen und hinteren Überlappungsbereiches vorteilhaft eine Verbundfestigkeit.

Für die Umsetzung der nicht vollflächigen Fixierung des Schrittabschnitts an Bauchabschnitt und/oder Rückenabschnitt wird auf die Ausführungen der EP2849696 B1 verwiesen.

Die Breite B10 der kleberfreien Längsstreifen, die Breite B12 der ersten Längskleberstreifen, die Breite B14 der randständigen Längskleberstreifen wird dabei verstanden als die maximale Erstreckung in Querrichtung, also quer zu deren Erstreckung in Längsrichtung.

Die Breite B16 der ersten Querkleberstreifen und die Breite B18 der randständigen Querkleberstreifen wird dabei verstanden als die maximale Erstreckung in Längsrichtung, also quer zu deren Erstreckung in Querrichtung.

Die Distanz D wird verstanden als der minimale Abstand und gemessen in Längsrichtung zwischen einem schrittzugewandten Rand des Rückenabschnitts und einem schrittzugewandten Rand des Bauchabschnitts.

Die maximale Breite B2 der Vlieslage ist die maximale Erstreckung der Vlieslage in Querrichtung.

An dieser Stelle sei ausgeführt, dass in der vorliegenden Erfindung jegliche Bemessungsangaben hinsichtlich Breite, Länge, Quererstreckung, Längserstreckung oder Abstand oder Distanz von Abschnitten in eben ausgebreitetem Zustand der den Inkontinenzartikel bildenden Flachmaterialien bestimmt werden, also gegebenenfalls nach Auftrennung der herstellerseitig schon angebrachten Seitennähte, so dass der betreffende Inkontinenzartikel in die in den Figuren beispielhaft dargestellte ebene Konfiguration gebracht werden kann, sofern kein anderer Hinweis auf einen davon abweichenden Zustand angeführt ist. Wenn der Inkontinenzartikel beispielsweise durch fadenförmige Elastifizierungsmittel im sogenannten "Stretchbond-Verfahren" elastifiziert wurde, so werden die Flächenmaterialien, wie in den Figuren angedeutet, derart ausgedehnt betrachtet, wie sie herstellerseitig als Flachmaterialien zugeführt werden oder nachträglich bis zu ihrer natürlichen Ausgangserstreckung ohne Elastifizierungsmittel ausgebreitet und auf eine ebene Fläche aufgelegt werden können. In dieser ebenen Fläche werden dann die Quererstreckungen, Längserstreckungen, Abmessungen, Abstände oder Distanzen ermittelt. Dieser Zustand ergibt sich bei undehnbaren Chassismaterialien auf Vliesbasis oder Folienbasis oder Vlies/Folien-Verbundbasis auf natürliche Weise.

Die Vlieslage weist vorzugsweise ein Flächengewicht von 7 - 20 g/m², weiter vorzugsweise von 7 - 18 g/m², weiter vorzugsweise von 7 - 15 g/m² auf.

Die Vlieslage kann dabei einlagig oder auch ein Laminat von mehreren Vliesstoffmateriallagen sein.

Die Vlieslage bildenden Materialien umfassen vorzugsweise Vliesstoffmaterialen wie Spinnvliese (S) oder Meltblownvliese (M) oder Kombinationen aus Spinnvlies (S) und Meltblownvlies (M), wie beispielsweise SM, SMS, SMMS, SMMMS. Auch Kardenvliese oder Air through bonded Kardenvliese sind alternativ möglich.

Das Backsheet-Material umfasst insbesondere eine Folie, insbesondere eine im Gebrauch flüssigkeitsdichte aber gleichwohl atmungsaktive, also wasserdampfdurchlässige Folie. Das Backsheet-Material weist insbesondere ein Flächengewicht von 12 - 25 g/m², weiter vorzugsweise von 13 - 23 g/m², weiter vorzugsweise von 14 - 20 g/m² auf.

Der Bauchabschnitt und/oder der Rückenabschnitt sind vorzugsweise in Querrichtung oder Hüftumfangsrichtung elastisch dehnbar ausgebildet. Vorzugsweise sind der Bauchabschnitt und/oder der Rückenabschnitt mit Elastifizierungsmittel ausgestattet. Als Elastifizierungsmittel können elastische Folien und/oder fadenförmige oder bandförmige Elastifizierungsmittel vorgesehen sein.

Dabei können vorzugsweise in einem schrittseitigen und den Beinöffnungen zugewandten Bereich des Bauchabschnitts und/oder des Rückenabschnitts erste Elastifizierungsmittel vorgesehen sein. Insbesondere ist vorgesehen, dass die ersten Elastifizierungsmittel im Bauchabschnitt und/oder im Rückenabschnitt sich ausgehend von den beiden Seitennahtbereichen in Richtung auf eine Längsmittelachse des Inkontinenzartikels erstrecken und dabei mit zunehmendem Abstand voneinander verlaufen.

Weiter vorzugsweise können im Bauchabschnitt und/oder im Rückenabschnitt in einem hüftöffnungszugewandten Bereich des Bauchabschnitts oder Rückenabschnitts zweite Elastifizierungsmittel vorgesehen sein, die sich in einem Abstand voneinander und parallel zueinander in Querrichtung oder Hüftumfangsrichtung erstrecken.

Der Inkontinenzartikel in Höschenform ist vorzugsweise ein Erwachsenen-Inkontinenzartikel.

Weitere Einzelheiten, Merkmale und Vorteile der Erfindung ergeben sich aus den beigefügten Patentansprüchen und aus der zeichnerischen Darstellung und nachfolgenden Beschreibung bevorzugter Ausführungsformen des erfindungsgemäßen Inkontinenzartikels.

In der Zeichnung zeigen:
- Figur 1: schematisch eine Draufsicht auf einen erfindungsgemäßen Inkontinenzartikel, wobei Bauchabschnitt, Rückenabschnitt und Schrittabschnitt noch nicht zur Bildung einer Höschenform verbunden sind, sondern in flachgelegtem und eben ausgebreitetem Zustand sind;
- Figur 2: schematisch eine Draufsicht auf einen weiteren erfindungsgemäßen Inkontinenzartikel in ausgebreiteter Konfiguration;
- Figur 3: schematisch eine Schnittansicht entlang der Linie I - I des Schrittabschnitts des Inkontinenzartikels aus Figur 1 oder Figur 2 mit eingezeichneten Abmessungen B10, B12, B14 und B2;
- Figur 4: schematisch eine Schnittansicht entlang der Linie II - II des Inkontinenzartikels aus Figur 1 oder 2 mit eingezeichneten Abmessungen L1 und L2;
- Figur 5: schematisch einen Schrittabschnitt, dabei mit der Fixierung der Vlieslage mit dem Backsheet-Material mittels Längskleberstreifen von gleicher Breite.
- Figur 6: schematisch einen Schrittabschnitt, dabei mit der Fixierung der Vlieslage mit dem Backsheet-Material mittels Längskleberstreifen von unterschiedlicher Breite.
- Figur 7: schematisch eine der Figur 1 entsprechende Darstellung, wobei die Fixierung des Schrittabschnitts mit dem Bauchabschnitt und Rückenabschnitt mittels Querkleberstreifen erkennbar ist;
- Figur 8: schematisch eine vergrößerte ausschnittsweise Darstellung im Bereich des Überlappungsbereiches von Schrittabschnitt und Bauchabschnitt des Inkontinenzartikels nach Figur 7, mit eingezeichneten Abmessungen B16 und B18; und
- Figur 9: schematisch eine der Figur 1 entsprechende Darstellung, wobei die Fixierung der Vlieslage mit dem Backsheet-Material mittels Längskleberstreifen und die Fixierung des Schrittabschnitts mit dem Bauchabschnitt und Rückenabschnitt mittels Querkleberstreifen erkennbar ist.

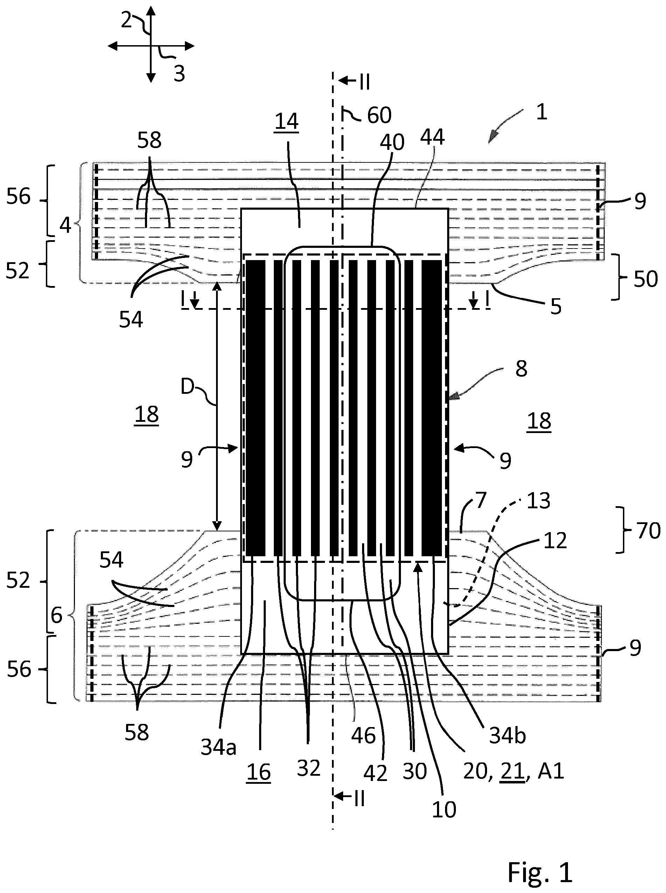

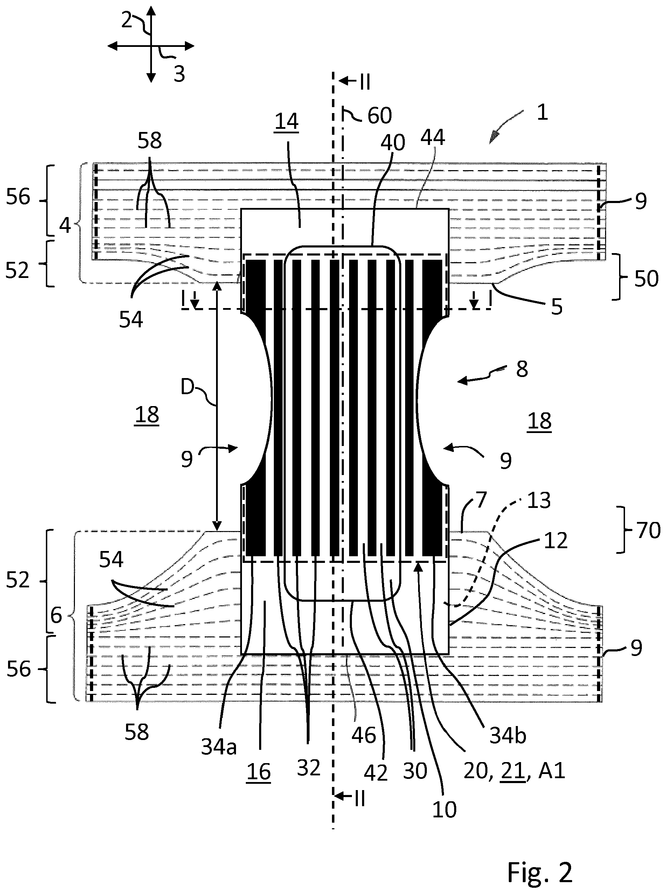

Die Figuren 1 und 2 zeigen insgesamt einen mit dem Bezugszeichen 1 bezeichneten Inkontinenzartikel in Höschenform für die Aufnahme von Körperausscheidungen, in dessen nicht an den Seitennähten verbundener und in plan ausgebreiteter Konfiguration.

Der Inkontinenzartikel 1 hat einen dreikomponentigen Aufbau, und zwar einen vorderen Bauchabschnitt 4 und einen hinteren Rückenabschnitt 6, die in der Längsrichtung 2 über die Distanz D voneinander beabstandet sind. Ein einen Absorptionskörper 10 aufweisender Schrittabschnitt 8, der sich in der Längsrichtung 2 zwischen Bauchabschnitt 4 und Rückenabschnitt 6 erstreckt, ist an den Bauchabschnitt 4 und an den Rückenabschnitt 6 in einem jeweiligen vorderen und hinteren Überlappungsbereich 14, 16 unlösbar angefügt. Zur Bildung der Höschenform und damit zur Bildung eines in Querrichtung 3 und in Hüftumfangsrichtung durchgehenden Bauch- und Rückenbands mit einer in Hüftumfangsrichtung geschlossenen Hüftöffnung werden Bauchabschnitt 4 und Rückenabschnitt 6 an beidseitigen Seitennahtbereichen 9 herstellerseitig miteinander verbunden, so dass der Schrittabschnitt 8, der Bauchabschnitt 4 und der Rückenabschnitt 6 gemeinsam die Beinöffnungen 18 des Inkontinenzartikels 1 begrenzen.

Vorteilhaft sind der Bauchabschnitt und der Rückenabschnitt in Querrichtung oder Hüftumfangsrichtung elastisch dehnbar ausgebildet. Vorzugsweise weisen dazu der Bauchabschnitt 4 und/oder der Rückenabschnitt 6 jeweils in einem schrittseitigen und den Beinöffnungen zugewandten Bereich 52 erste Elastifizierungsmittel 54 auf, die sich ausgehend von den beiden Seitennahtbereichen 9 in Richtung auf eine Längsmittelachse 60 des Inkontinenzartikels 1 erstrecken und dabei mit zunehmendem Abstand voneinander verlaufen. Im hüftöffnungszugewandten Bereich 56 des Bauchabschnitts 4 und Rückenabschnitts 6 sind insbesondere zweite Elastifizierungsmittel 58 vorgesehen, die sich in einem Abstand voneinander und parallel zueinander in Querrichtung oder Hüftumfangsrichtung 3 erstrecken.

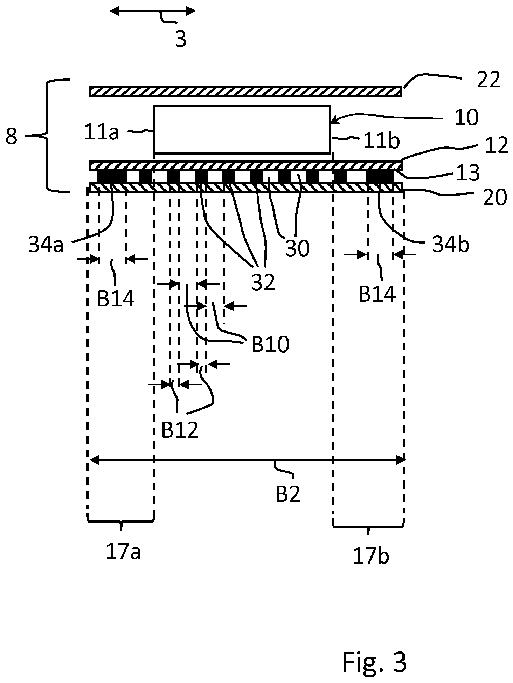

Der Schrittabschnitt 8 weist, wie auch in der Figur 3 als eine schematische Darstellung der Schnittansicht entlang der Linie I - I des Inkontinenzartikels aus Figur 1 oder Figur 2 dargestellt, ein Backsheet Material 12 auf, auf dessen körperabgewandter Oberseite 13 eine Vlieslage 20 vorgesehen ist. Die Vlieslage erstreckt sich dabei in der Längsrichtung 2 über die Distanz D zwischen Bauchabschnitt 4 und Rückenabschnitt 6 und erstreckt sich bis in den vorderen und hinteren Überlappungsbereich 14, 16 und überlappt dabei den Bauchabschnitt und den Rückenabschnitt. Auf der körperzugewandten Seite des Schrittabschnitts ist ein Topsheet-Material 22.

Die Vlieslage 20 ist mit dem Backsheet-Material 12 mittels eines ersten und eines zweiten in Längsrichtung 2 erstreckten randständigen Längskleberstreifens 34a, 34b und mittels einer Vielzahl bezüglich der randständigen Längksleberstreifen 34a, 34b in Querrichtung 3 zwischen diesen innenliegenden und in Längsrichtung 2 erstreckten, parallel zueinander und voneinander durch kleberfreie Längsstreifen 30 beabstandeten ersten Längskleberstreifen 32 verbunden.

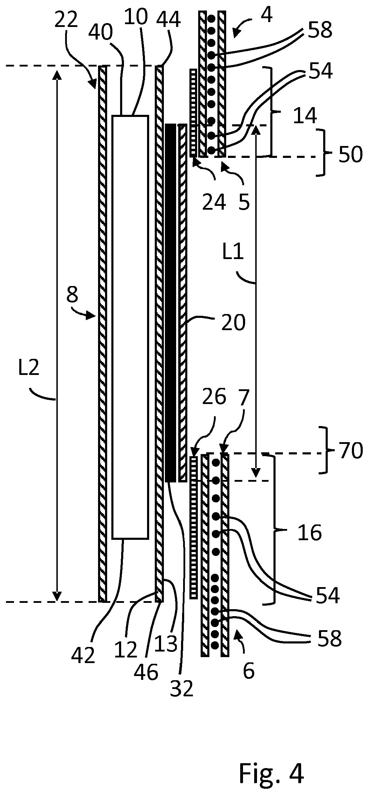

Die Vlieslage 20 endet in Längsrichtung 2 vor einem vorderen Ende 44 und/oder einem hinteren Ende 46 des Backsheet-Materials 12, wie in Figur 1, Figur 2 und auch Figur 4 dargestellt ist. So beträgt ein Verhältnis der Länge L1 der Vlieslage 20 in Längsrichtung 2 zur Länge L2 des Backsheet-Materials 12 in Längsrichtung 2, berechnet als L1/L2, vorzugsweise mindestens 0,5 und weiter vorzugsweise höchstens 0,9, weiter vorzugsweise höchstens 0,8, weiter vorzugsweise höchstens 0,7.

Vorzugsweise endet die Vlieslage 20 in Längsrichtung 2 vor einem vorderen Ende 40 und/oder einem hinteren Ende 42 des Absorptionskörpers 10, wie in Figur 1, Figur 2 und auch Figur 4 dargestellt ist.

Beim Schrittabschnitt 8 können die beidseitigen und die Beinöffnungen 18 begrenzenden Randbereiche 9 des Schrittabschnitts 8 in Längsrichtung 2 ausschließlich gerade verlaufen (Figur 1) oder zumindest entlang eines Teils der beidseitigen Randbereiche 9 bogenförmig konturiert verlaufen (Figur 2). Vorzugsweise erstrecken sich bei geraden Randbereichen 9 die randständigen Längskleberstreifen 34a, 34 zumindest über die Distanz D kontinuierlich, wie insbesondere auch einige der ersten Längskleberstreifen 32, weiter insbesondere alle ersten Längskleberstreifen 32. Im Falle einer bogenförmigen Konturierung der beidseitigen Randbereiche 9 des Schrittabschnitts 8 sind die randständigen Längskleberstreifen 34a, 34b und weiter insbesondere erste Längskleberstreifen 32, die den randständigen Längskleberstreifen unmittelbar benachbart angeordnet sind, im Bereich der Konturierung in Längsrichtung 2 unterbrochen.

Wie anhand der Figur 3 erkennbar, weisen die randständigen Längskleberstreifen 34a und 34b in Querrichtung 3 eine Breite B14 auf, die ersten Längskleberstreifen 32 eine Breite B12 und die kleberfreien Längsstreifen 30 eine Breite B10. Die Längsklebersteifen 32, 34a, 34b und die kleberfreien Längsstreifen 30 sind dabei vorzugsweise über die gesamte Erstreckung in Querrichtung der Vlieslage 20 mit einer Breite B2 angeordnet. Vorzugsweise erstrecken sich das Backsheet-Material 12 und die Vlieslage 20 außerhalb eines ersten und zweiten Längsrands 11a, 11b des Absorptionskörpers 10 unter Ausbildung eines sich in Querrichtung 3 erstreckenden ersten und zweiten Überhangs 17a, 17b. Der erste und zweite randständige Längskleberstreifen 34a, 34b sind innerhalb des jeweiligen ersten und zweiten Überhangs 17a, 17b und in einem Abstand zum jeweiligen Längsrand 11a, 11b des Absorptionskörpers angeordnet.

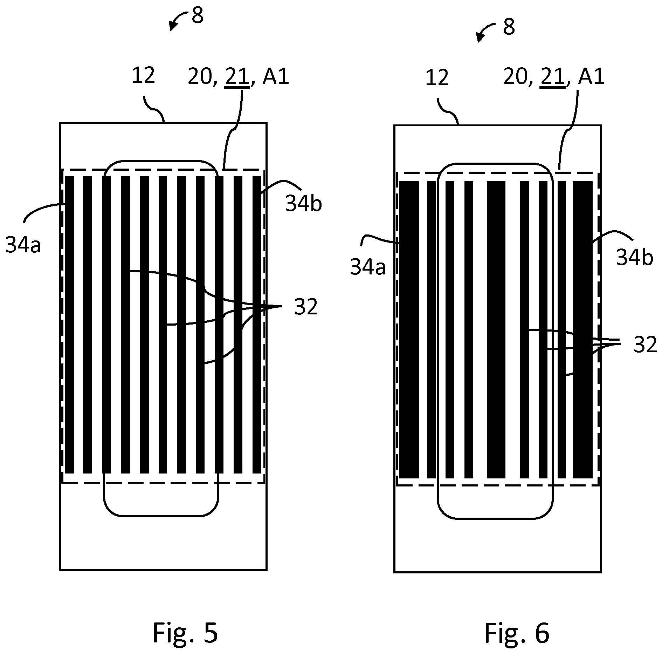

Für eine bessere Haftung zwischen dem Backsheet-Material und der Vlieslage, vor allem in den Randbereichen, wo die Lagen Gefahr laufen, voneinander abzuschälen, weisen der erste und zweite randständige Längskleberstreifen 34a, 34b vorzugsweise eine Breite B14 auf, die mit der Breite B12 der ersten Längskleberstreifen 32 zumindest übereinstimmt wie schematisch in der Figur 5 (darstellend einen Schrittabschnitt 8) angedeutet ist, oder insbesondere größer ist als die Breite B12 der ersten Längskleberstreifen 32.

Figur 6 (darstellend einen Schrittabschnitt 8) zeigt schematisch eine alternative Ausführung, bei der für die Fixierung der Vlieslage 20 zum Backsheet-Material 12 die Breite eines innersten ersten Längskleberstreifen größer ist als die Breite der übrigen ersten Längskleberstreifen 32 und dabei die Breite des innersten ersten Längskleberstreifens nicht größer ist als die Breite der randständigen Längskleberstreifen 34a, 34b.

In einer bevorzugten Ausführung der Figuren 1 oder 2 weisen die randständigen Längskleberstreifen 34a, 34b in Querrichtung 3 eine Breite B14 auf, die größer ist als eine Breite B12 der ersten Längskleberstreifen 32. Die Breite B14 ist dabei insbesondere größer ist als die Breite B12 der zu den randständigen Längskleberstreifen 34a, 34b unmittelbar benachbart angeordneten ersten Längskleberstreifens 32, weiter insbesondere größer als die Breite B12 von allen ersten Längskleberstreifen 32. Die Breite B14 beträgt vorzugsweise wenigstens das 1,5-fache, insbesondere wenigstens das 2-fache, und weiter insbesondere höchstens das 20-fache, insbesondere höchstens das 15-fache, insbesondere höchstens das 10-fache, insbesondere höchstens das 8-fache, weiter insbesondere höchstens das 5-fache, insbesondere höchstens das 4-fache der Breite B12 der ersten Längskleberstreifen 32.

Die Anordnung der ersten Längskleberstreifen 32 und der kleberfreien Längsstreifen 30 ist bevorzugt derart, dass ein Verhältnis der Breite B12 von ersten Längskleberstreifen 32 zur Breite B10 von unmittelbar benachbarten kleberfreien Längsstreifen 30, berechnet als B12/B10, insbesondere 0,2 - 3,0, insbesondere 0,5 - 2,5, weiter insbesondere 0,7- 2,5, weiter insbesondere 0,9 - 2,3, weiter insbesondere 1,0 - 2,0 beträgt.

Die Breite B12 der ersten Längskleberstreifen 32 und/oder die Breite B14 der randständigen Längskleberstreifen 34 sind vorzugsweise in Bezug auf die Breite B2 in Querrichtung der Vlieslage 20 abgestimmt, um die dem Tragekomfort positiv beisteuernde Eigenschaften wie Atmungsaktivität und Flexibilität beibehalten zu können. Ein Verhältnis der Breite B12 zur Breite B2, berechnet als B12/B2, beträgt vorzugsweise höchstens 0,10, vorzugsweise höchstens 0,08, weiter vorzugsweise höchstens 0,06, weiter vorzugsweise höchstens 0,04, weiter vorzugsweise höchstens 0,02. Ein Verhältnis der Breite B14 zur Breite B2, berechnet als B14/B2, beträgt 0,01 - 0,15, vorzugsweise 0,01 - 0,10, vorzugsweise 0,01 - 0,08, weiter vorzugsweise 0,01 - 0,06, weiter vorzugsweise 0,01 - 0,05, weiter vorzugsweise 0,01 - 0,04, weiter vorzugsweise 0,01 - 0,03.

Die ersten Längskleberstreifen 32 und/oder der randständigen Längskleberstreifen 34a, 34b weisen vorzugweise ein Flächengewicht an Kleber von wenigstens 0,5 g/m², insbesondere wenigstens 0,7 g/m², weiter insbesondere wenigstens 1 g/m², insbesondere wenigstens 1,2 g/m², und insbesondere höchstens 10 g/m², weiter insbesondere höchstens 8 g/m², weiter insbesondere höchstens 6 g/m2, weiter insbesondere höchstens 4 g/m2 auf.

Die randständigen Längskleberstreifen 34a, 34b erstrecken sich zumindest in Längsrichtung 2 in dem Maße, dass die randständigen Längskleberstreifen dabei in einem vorderen Übergangsbereich 50 des Schrittabschnitts 8, der sich in Längsrichtung 2 vor und hinter einem schrittzugewandtem Rand 5 des Bauchabschnitts 4 erstreckt, vorhanden sind und/oder die randständigen Längskleberstreifen in einem hinteren Übergangsbereich 70 des Schrittabschnitts 8, der sich in Längsrichtung 2 vor und hinter einem schrittzugewandten Rand 7 des Rückenabschnitts 6 erstreckt, vorgesehen sind. Vorzugsweise sind dabei auch die ersten Längskleberstreifen 32 in ihrer Erstreckung in dem vorderen Übergangsbereich 50 des Schrittabschnitts und/oder in dem hinteren Übergangsbereich 70 des Schrittabschnitts vorhanden.

Die Vlieslage 20 überfängt das Backsheet-Material 12 unter Bildung einer Überlappungszone 21, die eine Fläche A1 aufweist. Die ersten Längskleberstreifen 32 und die randständigen Längskleberstreifen 34a, 34b bilden in Summe einen Bedeckungsgrad, berechnet als Gesamtfläche der Längskleberstreifen 32, 34a, 34b bezogen auf die Fläche A1 der Überlappungszone 21, von mindestens 10%, weiter insbesondere mindestens 12%, weiter insbesondere mindestens 14%, weiter insbesondere mindestens 16 %, und weiter insbesondere höchstens 80%, weiter insbesondere höchstens 75%, weiter insbesondere höchstens 70%, weiter insbesondere höchstens 65%.

Der Schrittabschnitts 8 ist im vorderen Überlappungsbereich 14 an den Bauchabschnitt 4 und im hinteren Überlappungsbereich 16 an den Rückenabschnitt 6 unlösbar angefügt. In der Figur 4 als schematische Schnittansicht entlang der Linie II - II des Inkontinenzartikels aus Figur 1 bzw. aus Figur 2 ist der vordere Fügebereich bzw. der hintere Fügebereich mit dem Bezugszeichen 24 bzw. 26 angedeutet.

Der Schrittabschnitt ist dabei im vorderen und hinteren Überlappungsbereich mit Orientierung der körperabgewandten Oberseite des Backsheet-Material an einer körperzugewandten Oberseite des Bauchabschnitts und des Rückenabschnitts angefügt.

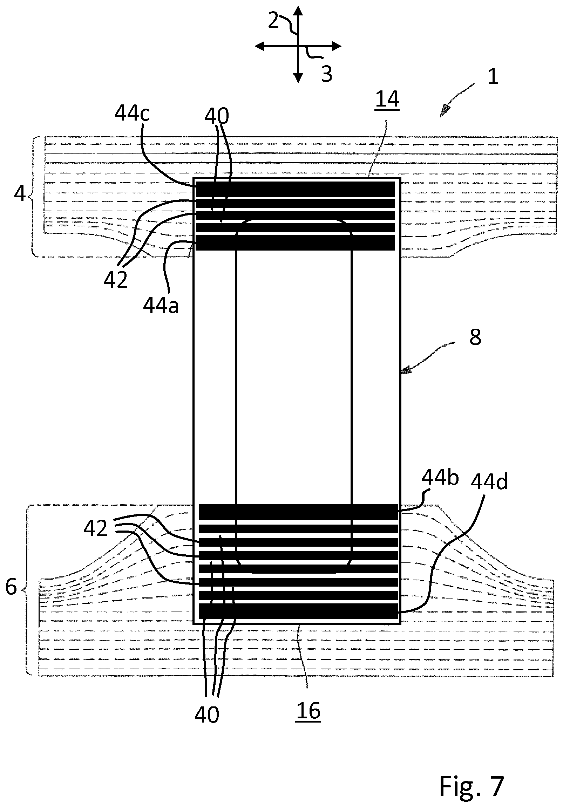

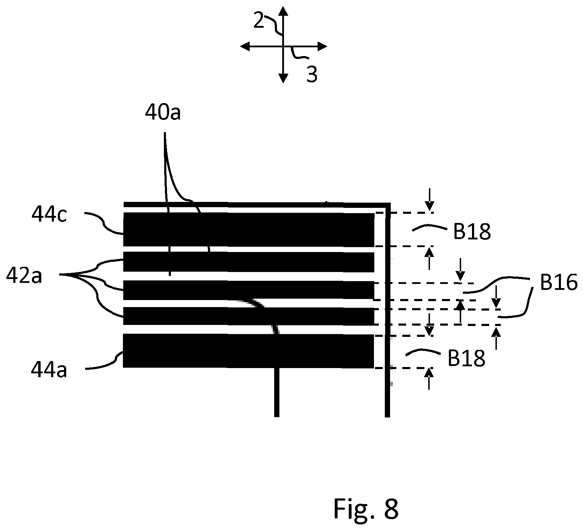

Für die unlösbare Fügung in den Fügebereichen 24, 26 wird vorzugsweise eine nicht vollflächige Verklebung gewählt, die anhand den Figuren 7 und 8 näher erläutert wird. Figur 7 zeigt dabei schematisch eine der Figur 1 entsprechende Darstellung, wobei eine bevorzugte Ausgestaltung des vorderen und hinteren Fügebereiches, nämlich die Fixierung des Schrittabschnitts 8 mit dem Bauchabschnitt 4 und mit dem Rückenabschnitt 6 mittels Querkleberstreifen erkennbar ist. Bevorzugt erfolgt die Fixierung mittels einer Vielzahl von im vorderen Überlappungsbereich 14 vorgesehener in Querrichtung 3 erstreckter parallel zueinander und voneinander durch kleberfreie Querstreifen 40 beabstandeten ersten Querkleberstreifen 42 und im hinteren Überlappungsbereich 16 mittels einer Vielzahl in Querrichtung 3 erstreckter parallel zueinander und voneinander durch kleberfreie Querstreifen 40 beabstandeten ersten Querkleberstreifen 42. Dabei sind in einer bevorzugten Ausführung in Querrichtung 3 erstreckte randständige Querkleberstreifen 44a, 44b, 44c, 44d vorgesehen, nämlich im vorderen und hinteren Fügebereich je ein schrittzugewandter erster randständiger Querkleberstreifen 44a, 44b und ein hüftöffnungszugewandter zweiter randständiger Querkleberstreifen 44c, 44d, die in Längsrichtung 2 eine Breite B18 aufweisen, die vorzugsweise größer ist als eine Breite B16 der bezüglich der randständigen Querkleberstreifen 44a, 44b, 44c, 44d innen liegenden ersten Querkleberstreifen 42, wie in Figur 8 als schematische vergrößerte ausschnittsweise Darstellung im Bereich des Überlappungsbereiches von Schrittabschnitt und Bauchabschnitt des Inkontinenzartikels nach Figur 7 dargestellt ist.

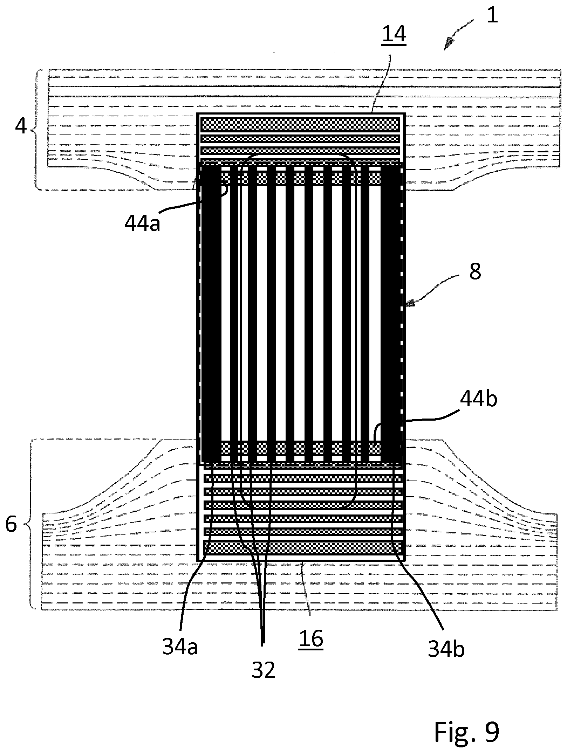

Figur 9 zeigt schematisch eine der Figur 1 entsprechende Darstellung, wobei die Fixierung der Vlieslage mit dem Backsheet-Material mittels Längskleberstreifen und die Fixierung des Schrittabschnitts 8 in den entsprechenden Überlappungsbereichen 14 und 16 mit dem Bauchabschnitt 4 und Rückenabschnitt 6 mittels Querkleberstreifen erkennbar ist.

In einer bevorzugten Ausführung, wie in Figur 9 dargestellt, überlappen in einem schrittzugewandten Randbereich des vorderen Überlappungsbereichs 14 und in einem schrittzugewandten Randbereich des hinteren Überlappungsbereichs 16 zumindest der erste und zweite randständige Längskleberstreifen 34a, 34b und weiter insbesondere einige der ersten Längskleberstreifen 32, weiter insbesondere alle ersten Längskleberstreifen mit einem schrittzugewandten ersten randständigen Querkleberstreifen 44a, 44b.

Mit dieser Überlappung von randständigen Längskleberstreifen und von randständigen Querkleberstreifen ergeben sich bei einem gattungsgemäßen dreikomponentigen Modulaufbau des Inkontinenzartikels und den damit einhergehenden möglichen Schwachstellen in den Übergangsbereichen im Verlauf der die Beinöffnung begrenzenden Ränder durch den Eintrag von randständigem Klebermaterial vorteilhaft verstärkte Bereiche.

Der sich ergebende gitterförmige Kleberauftrag aus der alternierenden Abfolge von Längskleberstreifen und kleberfreien Längsstreifen auf der dem Backsheet-Material zugewandten Oberseite der Vlieslage und den Querkleberstreifen als Fügebereichskleber zwischen dem Bauch- bzw. Rückenabschnitt und der dem Backsheet-Material abgewandten Oberseite des Vlieslage ergibt in den schrittzugewandten Randbereichen des vorderen und hinteren Überlappungsbereiches vorteilhaft eine Verbundfestigkeit.

## Patentansprüche

1. Absorbierender Inkontinenzartikel (1) in Höschenform für die Aufnahme von Körperausscheidungen umfassend einen vorderen Bauchabschnitt (4) und einen hinteren Rückenabschnitt (6), die in einer Längsrichtung (2) über eine Distanz D voneinander beabstandet sind und die zur Bildung eines in Quer- oder Hüftumfangsrichtung (3) durchgehenden Bauch- und Rückenbands mit einer in Hüftumfangsrichtung geschlossenen Hüftöffnung an beidseitigen Seitennahtbereichen (9) herstellerseitig miteinander verbunden sind, und einen Schrittabschnitt (8), der sich in der Längsrichtung (2) zwischen Bauchabschnitt (4) und Rückenabschnitt (6) erstreckt und an den Bauchabschnitt (4) in einem vorderen Überlappungsbereich (14) und an den Rückenabschnitt (6) in einem hinteren Überlappungsbereich (16) unlösbar angefügt ist, wobei der Schrittabschnitt (8) einen Absorptionskörper (10) und ein flüssigkeitsundurchlässiges Backsheet-Material (12) aufweist, und wobei der Schrittabschnitt (8), der Bauchabschnitt (4) und der Rückenabschnitt (6) Beinöffnungen (18) des Inkontinenzartikels (1) begrenzen, wobei auf einer körperabgewandten Oberseite (13) des Backsheet-Materials (12) eine in Längsrichtung (2) entlang der Distanz D und sich bis in den vorderen und hinteren Überlappungsbereich (14, 16) erstreckende Vlieslage (20) vorgesehen ist, wobei die Vlieslage (20) in Längsrichtung (2) vor einem vorderen Ende (44) des Backsheet-Materials (12) und/oder vor einem hinteren Ende (46) des Backsheet-Materials (12) endet, **dadurch gekennzeichnet, dass** die Vlieslage (20) mittels eines ersten und eines zweiten in Längsrichtung (2) erstreckten randständigen Längskleberstreifens (34a, 34b) und mittels einer Vielzahl bezüglich der randständigen Längskleberstreifen (34a, 34b) in Querrichtung (3) zwischen den randständigen Längskleberstreifen (34a, 34b) innenliegenden und in Längsrichtung (2) erstreckten, parallel zueinander und voneinander durch kleberfreie Längsstreifen (30) beabstandeten ersten Längskleberstreifen (32) mit dem Backsheet-Material (12) verbunden ist, wobei in Querrichtung (3) die randständigen Längskleberstreifen (34a, 34b) eine Breite B14, die ersten Längskleberstreifen (32) eine Breite B12 und die kleberfreien Längsstreifen eine Breite B10 aufweisen.

2. Absorbierender Inkontinenzartikel (1) nach Anspruch 1, **dadurch gekennzeichnet, dass** außerhalb eines ersten und zweiten Längsrands (11a, 11b) des Absorptionskörpers (10) das Backsheet-Material (12) und die Vlieslage (20) einen sich in Querrichtung (3) erstreckenden ersten und zweiten Überhang (17a, 17b) aufweisen und wobei der erste und zweite randständige Längskleberstreifen (34a, 34b) innerhalb des jeweiligen ersten und zweiten Überhangs (17a, 17b) und in einem Abstand zum jeweiligen Längsrand (11a, 11b) des Absorptionskörpers (10) angeordnet sind.

3. Absorbierender Inkontinenzartikel (1) nach Anspruch 1 oder 2, **dadurch gekennzeichnet, dass** beidseitige und die Beinöffnungen (18) begrenzende Randbereiche (9) des Schrittabschnitts (8) in Längsrichtung (2) ausschließlich gerade oder zumindest entlang eines Teils der beidseitigen Randbereiche (9) des Schrittabschnitts (8) bogenförmig konturiert verlaufen, wobei im Falle ausschließlich gerader Randbereiche (9) des Schrittabschnitts (8) die randständigen Längskleberstreifen (34a, 34b) sich zumindest über die Distanz D kontinuierlich erstrecken und weiter insbesondere einige der ersten Längskleberstreifen (32), weiter insbesondere alle ersten Längskleberstreifen (32) sich zumindest über die Distanz D kontinuierlich erstrecken oder im Falle bogenförmiger Konturierung der beidseitigen Randbereiche (9) des Schrittabschnitts (8) die randständigen Längskleberstreifen (34a, 34b) und insbesondere erste Längskleberstreifen (32), die den randständigen Längskleberstreifen (34a, 34b) unmittelbar benachbart angeordnet sind, im Bereich der Konturierung in Längsrichtung (2) unterbrochen sind.

4. Absorbierender Inkontinenzartikel (1) nach einem der vorstehenden Ansprüche, **dadurch gekennzeichnet, dass** die randständigen Längskleberstreifen (34a, 34b) sich in Längsrichtung (2) derart erstrecken, dass die randständigen Längskleberstreifen (34a, 34b) in einem vorderen Übergangsbereich (50) des Schrittabschnitts (8), der sich in Längsrichtung (2) vor und hinter einem schrittzugewandtem Rand (5) des Bauchabschnitts (4) erstreckt, vorhanden sind und dass die randständigen Längskleberstreifen (34a, 34b) in einem hinteren Übergangsbereich (70) des Schrittabschnitts (8), der sich in Längsrichtung (2) vor und hinter einem schrittzugewandten Rand (7) des Rückenabschnitts (6) erstreckt, vorhanden sind.

5. Absorbierender Inkontinenzartikel (1) nach einem der vorstehenden Ansprüche, **dadurch gekennzeichnet, dass** ein Verhältnis der Breite B12 von ersten Längskleberstreifen (32) zur Breite B10 von unmittelbar benachbarten kleberfreien Längsstreifen (30), berechnet als B12/B10, 0,2 - 3,0, insbesondere 0,5 - 2,5, weiter insbesondere 0,7- 2,5, weiter insbesondere 0,9 - 2,3 weiter insbesondere 1,0 - 2,0 beträgt.

6. Absorbierender Inkontinenzartikel (1) nach einem der vorstehenden Ansprüche, **dadurch gekennzeichnet, dass** die Breite B12 der ersten Längskleberstreifen (32) wenigstens 1 mm bis höchstens 10 mm, insbesondere bis höchstens 8 mm, weiter insbesondere bis höchstens 6 mm, weiter insbesondere bis höchstens 4 mm beträgt und/oder dass die Breite B10 der kleberfreien Längsstreifen (30) wenigstens 1 mm bis höchstens 12 mm, insbesondere bis höchstens 10 mm, insbesondere bis höchstens 8 mm, weiter insbesondere bis höchstens 6 mm, weiter insbesondere bis höchstens 4 mm beträgt.

7. Absorbierender Inkontinenzartikel (1) nach einem der vorstehenden Ansprüche, **dadurch gekennzeichnet, dass** die Breite B12 der ersten Längskleberstreifen (32) jeweils gleich groß ist und/oder die Breite B10 der kleberfreien Längsstreifen (30) jeweils gleich groß ist.

8. Absorbierender Inkontinenzartikel (1) nach einem der vorstehenden Ansprüche, **dadurch gekennzeichnet, dass** die Breite B14 der randständigen Längskleberstreifen (34a, 34b) größer ist als die Breite B12 der ersten Längskleberstreifen (32), insbesondere größer ist als die Breite B12 der zu den randständigen Längskleberstreifen (34a, 34b) unmittelbar benachbart angeordneten ersten Längskleberstreifen (32), weiter insbesondere größer ist als die Breite B12 von allen ersten Längskleberstreifen (32).

9. Absorbierender Inkontinenzartikel (1) nach Anspruch 8, **dadurch gekennzeichnet, dass** die Breite B14 der randständigen Längskleberstreifen (34a, 34b) dabei wenigstens das 1,5-fache, weiter insbesondere wenigstens das 2-fache und weiter insbesondere höchstens das 20-fache, insbesondere höchstens das 15-fache, insbesondere höchstens das 10-fache, insbesondere höchstens das 8-fache, weiter insbesondere höchstens das 5-fache, insbesondere höchstens das 4-fache der Breite B12 der ersten Längskleberstreifen (32) beträgt.

10. Absorbierender Inkontinenzartikel (1) nach einem der vorstehenden Ansprüche, **dadurch gekennzeichnet, dass** die Breite B14 der randständigen Längskleberstreifen (34a, 34b) wenigstens 3 mm bis höchstens 20 mm, insbesondere bis höchstens 15 mm, weiter insbesondere bis höchstens 12 mm, weiter insbesondere bis höchstens 10 mm, weiter insbesondere bis höchstens 8 mm, weiter insbesondere bis höchstens 6 mm beträgt.

11. Absorbierender Inkontinenzartikel (1) nach einem der vorstehenden Ansprüche, **dadurch gekennzeichnet, dass** die Vlieslage (20) in Querrichtung (3) eine maximale Breite B2 aufweist und ein Verhältnis der Breite B12 der ersten Längskleberstreifen (32) zur Breite B2, berechnet als B12/B2, höchstens 0,10, vorzugsweise höchstens 0,08, weiter vorzugsweise höchstens 0,06, weiter vorzugsweise höchstens 0,04, weiter vorzugsweise höchstens 0,02 beträgt und/oder dass die Vlieslage (20) in Querrichtung (3) eine maximale Breite B2 aufweist und ein Verhältnis der Breite B14 der randständigen Längskleberstreifen (34a, 34b) zur Breite B2, berechnet als B14/B2, 0,01 - 0,15, vorzugsweise 0,01 - 0,10, vorzugsweise 0,01 - 0,08, weiter vorzugsweise 0,01 - 0,06 ,weiter vorzugsweise 0,01 - 0,05, weiter vorzugsweise 0,01 - 0,04, weiter vorzugsweise 0,01 - 0,03 beträgt.

12. Absorbierender Inkontinenzartikel (1) nach einem der vorstehenden Ansprüche, **dadurch gekennzeichnet, dass** die Vlieslage (20) in Längsrichtung (2) eine Länge L1 aufweist und das Backsheet-Material (12) in Längsrichtung (2) eine Länge L2 aufweist und ein Verhältnis der Länge L1 zu Länge L2, berechnet als L1/L2, mindestens 0,5 und weiter vorzugsweise höchstens 0,9, weiter vorzugsweise höchstens 0,8, weiter vorzugsweise höchstens 0,7 beträgt.

13. Absorbierender Inkontinenzartikel (1) nach einem der vorstehenden Ansprüche, **dadurch gekennzeichnet, dass** die Vlieslage (20) in Längsrichtung (2) vor einem vorderen Ende (40) des Absorptionskörpers (10) und/oder vor einem hinteren Ende (42) des Absorptionskörpers (10) endet.

14. Absorbierender Inkontinenzartikel (1) nach einem der vorstehenden Ansprüche, **dadurch gekennzeichnet, dass** die ersten Längskleberstreifen (32) und/oder die randständigen Längskleberstreifen (34a, 34b) ein Flächengewicht an Kleber von wenigstens 0,5 g/m², insbesondere wenigstens 0,7 g/m², weiter insbesondere wenigstens 1 g/m², weiter insbesondere wenigstens 1,2 g/m², und insbesondere höchstens 10 g/m², weiter insbesondere höchstens 8 g/m², weiter insbesondere höchstens 6 g/m², weiter insbesondere höchstens 4 g/m² aufweisen.

15. Absorbierender Inkontinenzartikel (1) nach einem der vorstehenden Ansprüche, **dadurch gekennzeichnet, dass** die randständigen Längskleberstreifen (34a, 34b) ein höheres Flächengewicht an Kleber aufweisen als die ersten Längskleberstreifen (32), insbesondere das Flächengewicht mindestens das 2-fache, insbesondere mindestens das 3-fache, weiter insbesondere mindestens das 4-fache, weiter insbesondere mindestens das 5-fache und weiter insbesondere höchstens das 10-fache des Flächengewichts an Kleber der ersten Längskleberstreifen (32) beträgt.

16. Absorbierender Inkontinenzartikel (1) nach einem der vorstehenden Ansprüche, **dadurch gekennzeichnet, dass** die Vlieslage (20) das Backsheet-Material (12) unter Bildung einer Überlappungszone (21) überfängt, wobei die Überlappungszone (21) eine Fläche A1 aufweist, und wobei die ersten Längskleberstreifen (32) und die randständigen Längskleberstreifen (34a, 34b) in Summe einen Bedeckungsgrad, berechnet als Gesamtfläche der Längskleberstreifen (32, 34a, 34b) bezogen auf die Fläche A1 der Überlappungszone (21), von mindestens 10%, weiter insbesondere mindestens 12%, weiter insbesondere mindestens 14%, weiter insbesondere mindestens 16 %, und weiter insbesondere höchstens 80%, weiter insbesondere höchstens 75%, weiter insbesondere höchstens 70%, weiter insbesondere höchstens 65% aufweisen.

17. Absorbierender Inkontinenzartikel (1) nach einem der vorstehenden Ansprüche, **dadurch gekennzeichnet, dass** die Vlieslage (20) das Backsheet-Material (12) in Querrichtung (3) vollständig überfängt.

18. Absorbierender Inkontinenzartikel (1) nach einem der vorstehenden Ansprüche, **dadurch gekennzeichnet, dass** der Schrittabschnitt (8) an den Bauchabschnitt (4) mittels einer Vielzahl von im vorderen Überlappungsbereich (14) vorgesehener in Querrichtung (3) erstreckter parallel zueinander und voneinander durch kleberfreie Querstreifen (40) beabstandeter erster Querkleberstreifen (42), die bezüglich optionaler randständiger Querkleberstreifen (44a, 44c) innen liegen, verbunden ist und/oder der Schrittabschnitt (8) an den Rückenabschnitt (6) mittels einer Vielzahl von im hinteren Überlappungsbereich (16) vorgesehener in Querrichtung (3) erstreckter parallel zueinander und voneinander durch kleberfreie Querstreifen (40) beabstandeter erster Querkleberstreifen (42), die bezüglich optionaler randständiger Querkleberstreifen (44b, 44d) innen liegen, verbunden ist.

19. Absorbierender Inkontinenzartikel (1) nach Anspruch 18, **dadurch gekennzeichnet, dass** im vorderen Überlappungsbereich (14) und/oder im hinteren Überlappungsbereich (16) in Querrichtung (3) erstreckte randständige Querkleberstreifen (44a, 44b, 44c, 44d) vorgesehen sind, die in Längsrichtung (2) eine Breite B18 aufweisen, wobei die Breite B18 größer ist als eine Breite B16 der ersten Querkleberstreifen (42) und/oder dass im vorderen Überlappungsbereich (14) und/oder im hinteren Überlappungsbereich (16) ein in Querrichtung (3) erstreckter schrittzugewandter erster randständiger Querkleberstreifen (44a, 44b) und ein in Querrichtung (3) erstreckter hüftöffnungszugewandter zweiter randständiger Querkleberstreifen (44c, 44d) und in Längsrichtung (2) zwischen diesen eine Vielzahl von innenliegenden ersten Querkleberstreifen (42) vorgesehen sind, wobei eine Breite B18 der randständigen Querkleberstreifen (44a, 44b, 44c, 44d) größer ist als eine Breite B16 der bezüglich der randständigen Querkleberstreifen (44a, 44b, 44c, 44d) innenliegenden ersten Querkleberstreifen (42), und insbesondere die Breite B18 dabei wenigstens das 4-fache, insbesondere wenigstens das 5-fache, und weiter insbesondere höchstens das 8-fache, insbesondere höchstens das 7-fache der Breite B16 der innenliegenden ersten Querkleberstreifen (42) beträgt.

20. Absorbierender Inkontinenzartikel (1) nach einem der vorstehenden Ansprüche 18 - 19, **dadurch gekennzeichnet, dass** in einem schrittzugewandten Randbereich des vorderen Überlappungsbereichs (14) und/oder in einem schrittzugewandten Randbereich des hinteren Überlappungsbereichs (16) zumindest der erste und zweite randständige Längskleberstreifen (34a, 34b) und weiter insbesondere einige der ersten Längskleberstreifen (32), weiter insbesondere alle ersten Längskleberstreifen (32) mit einem schrittzugewandten ersten randständigen Querkleberstreifen (44a, 44b) überlappen.

## Claims

1. Absorbent incontinence product (1) in brief form for the absorption of bodily excretions, comprising a front abdominal portion (4) and a rear back portion (6) which in a longitudinal direction (2) are spaced apart from one another over a distance D and which are joined to one another at the manufacturer end at bilateral side seam regions (9) to form an abdominal and back band which is continuous in transverse or hip girth direction (3), said band having a hip opening which is closed in hip girth direction, and comprising a crotch portion (8) which extends in the longitudinal direction (2) between abdominal portion (4) and back portion (6) and is indissolubly attached to the abdominal portion (4) in a front overlap region (14) and to the back portion (6) in a rear overlap region (16), wherein the crotch portion (8) comprises an absorption element (10) and a fluid-impervious backsheet material (12), and wherein the crotch portion (8), the abdominal portion (4) and the back portion (6) delimit leg openings (18) in the incontinence product (1), wherein a nonwoven ply (20) which extends in longitudinal direction (2) along the distance D and into the front and rear overlap regions (14, 16) is provided on a distal top side (13) of the backsheet material (12), wherein the nonwoven ply (20) in longitudinal direction (2) ends before a front end (44) of the backsheet material (12) and/or before a rear end (46) of the backsheet material (12), **characterized in that** the nonwoven ply (20) is joined to the backsheet material (12) by means of first and second marginal longitudinal adhesive strips (34a, 34b), which are extended in longitudinal direction (2), and by means of a multiplicity of first longitudinal adhesive strips (32) which are interior in relation to the marginal longitudinal adhesive strips (34a, 34b) in transverse direction (3) between the marginal longitudinal adhesive strips (34a, 34b) and which are extended in longitudinal direction (2) and which are spaced apart parallel to one another and from one another by adhesive-free longitudinal strips (30), wherein in transverse direction (3) the marginal longitudinal adhesive strips (34a, 34b) have a width B14, the first longitudinal adhesive strips (32) have a width B12 and the adhesive-free longitudinal strips have a width B10.

2. Absorbent incontinence product (1) according to Claim 1, **characterized in that** outside of first and second longitudinal margins (11a, 11b) of the absorption element (10), the backsheet material (12) and the nonwoven ply (20) have first and second overhangs (17a, 17b) which extend in transverse direction (3), and wherein the first and second marginal longitudinal adhesive strips (34a, 34b) are disposed within the respective first and second overhangs (17a, 17b) and at a spacing apart from the respective longitudinal margin (11a, 11b) of the absorption element (10).

3. Absorbent incontinence product (1) according to Claim 1 or 2, **characterized in that** bilateral marginal regions (9) of the crotch portion (8) which delimit the leg openings (18) run in longitudinal direction (2) exclusively linearly or with arcuate contouring at least along a part of the bilateral marginal regions (9) of the crotch portion (8), wherein, in the case of exclusively linear marginal regions (9) of the crotch portion (8), the marginal longitudinal adhesive strips (34a, 34b) extend continuously at least over the distance D and, further in particular, some of the first longitudinal adhesive strips (32), further in particular all the first longitudinal adhesive strips (32), extend continuously at least over the distance D or, in the case of arcuate contouring of the bilateral marginal regions (9) of the crotch portion (8), the marginal longitudinal adhesive strips (34a, 34b) and, in particular, first longitudinal adhesive strips (32) which are disposed directly adjacent to the marginal longitudinal adhesive strips (34a, 34b) are interrupted in longitudinal direction (2) in the region of the contouring.

4. Absorbent incontinence product (1) according to any of the preceding claims, **characterized in that** the marginal longitudinal adhesive strips (34a, 34b) extend in longitudinal direction (2) in such a way that the marginal longitudinal adhesive strips (34a, 34b) are present in a front transition region (50) of the crotch portion (8), which extends in longitudinal direction (2) before and behind a crotch-facing margin (5) of the abdominal portion (4) and **in that** the marginal longitudinal adhesive strips (34a, 34b) are present in a rear transition region (70) of the crotch portion (8), which extends in longitudinal direction (2) before and behind a crotch-facing margin (7) of the back portion (6).

5. Absorbent incontinence product (1) according to any of the preceding claims, **characterized in that** a ratio of the width B12 of first longitudinal adhesive strips (32) to the width B10 of directly adjacent, adhesive-free longitudinal strips (30), calculated as B12/B10, is 0.2 - 3.0, in particular 0.5 - 2.5, further in particular 0.7 - 2.5, further in particular 0.9 - 2.3, further in particular 1.0 - 2.0.

6. Absorbent incontinence product (1) according to any of the preceding claims, **characterized in that** the width B12 of the first longitudinal adhesive strips (32) is at least 1 mm to at most 10 mm, in particular to at most 8 mm, further in particular to at most 6 mm, further in particular to at most 4 mm and/or **in that** the width B10 of the adhesive-free longitudinal strips (30) is at least 1 mm to at most 12 mm, in particular to at most 10 mm, in particular to at most 8 mm, further in particular to at most 6 mm, further in particular to at most 4 mm.

7. Absorbent incontinence product (1) according to any of the preceding claims, **characterized in that** the width B12 of the first longitudinal adhesive strips (32) is the same in each case and/or the width B10 of the adhesive-free longitudinal strips (30) is the same in each case.

8. Absorbent incontinence product (1) according to any of the preceding claims, **characterized in that** the width B14 of the marginal longitudinal adhesive strips (34a, 34b) is greater than the width B12 of the first longitudinal adhesive strips (32), in particular is greater than the width B12 of the first longitudinal adhesive strips (32) disposed directly adjacent to the marginal longitudinal adhesive strips (34a, 34b), further in particular is greater than the width B12 of all the first longitudinal adhesive strips (32).

9. Absorbent incontinence product (1) according to Claim 8, **characterized in that** the width B14 of the marginal longitudinal adhesive strips (34a, 34b) here is at least 1.5 times, further in particular at least 2 times and further in particular at most 20 times, in particular at most 15 times, in particular at most 10 times, in particular at most 8 times, further in particular at most 5 times, in particular at most 4 times the width B12 of the first longitudinal adhesive strips (32).

10. Absorbent incontinence product (1) according to any of the preceding claims, **characterized in that** the width B14 of the marginal longitudinal adhesive strips (34a, 34b) is at least 3 mm to at most 20 mm, in particular to at most 15 mm, further in particular to at most 12 mm, further in particular to at most 10 mm, further in particular to at most 8 mm, further in particular to at most 6 mm.

11. Absorbent incontinence product (1) according to any of the preceding claims, **characterized in that** the nonwoven ply (20) in transverse direction (3) has a maximum width B2 and a ratio of the width B12 of the first longitudinal adhesive strips (32) to the width B2, calculated as B12/B2, is at most 0.10, preferably at most 0.08, further preferably at most 0.06, further preferably at most 0.04, further preferably at most 0.02 and/or **in that** the nonwoven ply (20) in transverse direction (3) has a maximum width B2 and a ratio of the width B14 of the marginal longitudinal adhesive strips (34a, 34b) to the width B2, calculated as B14/B2, is 0.01 - 0.15, preferably 0.01 - 0.10, preferably 0.01 - 0.08, further preferably 0.01 - 0.06, further preferably 0.01 - 0.05, further preferably 0.01 - 0.04, further preferably 0.01 - 0.03.

12. Absorbent incontinence product (1) according to any of the preceding claims, **characterized in that** the nonwoven ply (20) in longitudinal direction (2) has a length L1 and the backsheet material (12) in longitudinal direction (2) has a length L2, and a ratio of the length L1 to length L2, calculated as L1/L2, is at least 0.5 and further preferably at most 0.9, further preferably at most 0.8, further preferably at most 0.7.

13. Absorbent incontinence product (1) according to any of the preceding claims, **characterized in that** the nonwoven ply (20) in longitudinal direction (2) ends before a front end (40) of the absorption element (10) and/or before a rear end (42) of the absorption element (10).

14. Absorbent incontinence product (1) according to any of the preceding claims, **characterized in that** the first longitudinal adhesive strips (32) and/or the marginal longitudinal adhesive strips (34a, 34b) have a surface weight of adhesive of at least 0.5 g/m², in particular at least 0.7 g/m², further in particular at least 1 g/m², further in particular at least 1.2 g/m², and in particular at most 10 g/m², further in particular at most 8 g/m², further in particular at most 6 g/m², further in particular at most 4 g/m².

15. Absorbent incontinence product (1) according to any of the preceding claims, **characterized in that** the marginal longitudinal adhesive strips (34a, 34b) have a higher surface weight of adhesive than the first longitudinal adhesive strips (32), the surface weight being in particular at least 2 times, in particular at least 3 times, further in particular at least 4 times, further in particular at least 5 times and further in particular at most 10 times the surface weight of adhesive of the first longitudinal adhesive strips (32).

16. Absorbent incontinence product (1) according to any of the preceding claims, **characterized in that** the nonwoven ply (20) overlies the backsheet material (12) to form an overlap zone (21), wherein the overlap zone (21) has an area A1, and wherein the first longitudinal adhesive strips (32) and the marginal longitudinal adhesive strips (34a, 34b) in total have a coverage, calculated as total area of the longitudinal adhesive strips (32, 34a, 34b) relative to the area A1 of the overlap zone (21), of at least 10%, further in particular at least 12%, further in particular at least 14%, further in particular at least 16%, and further in particular at most 80%, further in particular at most 75%, further in particular at most 70%, further in particular at most 65%.

17. Absorbent incontinence product (1) according to any of the preceding claims, **characterized in that** the nonwoven ply (20) overlies the backsheet material (12) completely in transverse direction (3).

18. Absorbent incontinence product (1) according to any of the preceding claims, **characterized in that** the crotch portion (8) is connected to the abdominal portion (4) by means of a multiplicity of first transverse adhesive strips (42), which are provided in the front overlap region (14) and which are extended in transverse direction (3) and which are spaced apart parallel to one another and from one another by adhesive-free transverse strips (40) and which lie on the inside in relation to optional marginal transverse adhesive strips (44a, 44c), and/or the crotch portion (8) is connected to the back portion (6) by means of a multiplicity of first transverse adhesive strips (42), which are provided in the rear overlap region (16) and which are extended in transverse direction (3) and which are spaced apart parallel to one another and from one another by adhesive-free transverse strips (40) and which lie on the inside in relation to optional marginal transverse adhesive strips (44b, 44d).

19. Absorbent incontinence product (1) according to Claim 18, **characterized in that** in the front overlap region (14) and/or in the rear overlap region (16), marginal transverse adhesive strips (44a, 44b, 44c, 44d) are provided which are extended in transverse direction (3) and which in longitudinal direction (2) have a width B18, wherein the width B18 is greater than a width B16 of the first transverse adhesive strips (42) and/or **in that** in the front overlap region (14) and/or in the rear overlap region (16), a crotch-facing first marginal transverse adhesive strip (44a, 44b) extended in transverse direction (3), a hip opening-facing second marginal transverse adhesive strip (44c, 44d) extended in transverse direction (3) and, in longitudinal direction (2) between these strips, a multiplicity of interior first transverse adhesive strips (42) are provided, wherein a width B18 of the marginal transverse adhesive strips (44a, 44b, 44c, 44d) is greater than a width B16 of the first transverse adhesive strips (42) which are interior in relation to the marginal transverse adhesive strips (44a, 44b, 44c, 44d), and in particular the width B18 here is at least 4 times, in particular at least 5 times, and further in particular at most 8 times, in particular at most 7 times, the width B16 of the interior first transverse adhesive strips (42).

20. Absorbent incontinence product (1) according to any of preceding Claims 18-19, **characterized in that** in a crotch-facing marginal region of the front overlap region (14) and/or in a crotch-facing marginal region of the rear overlap region (16), at least the first and second marginal longitudinal adhesive strips (34a, 34b) and further in particular some of the first longitudinal adhesive strips (32), further in particular all the first longitudinal adhesive strips (32), overlap with a crotch-facing first marginal transverse adhesive strip (44a, 44b).

## Revendications

1. Article absorbant pour l'incontinence (1) sous forme de culotte destiné à absorber des excrétions corporelles, comprenant une section abdominale avant (4) et une section dorsale arrière (6), qui sont espacées l'une de l'autre, dans une direction longitudinale (2), d'une distance D et qui sont reliées l'une à l'autre, côté fabrication, au niveau de zones de couture latérales (9) situées des deux côtés, afin de former une ceinture abdominale et dorsale continue dans une direction transversale ou de circonférence de hanche (3), avec une ouverture de hanche fermée dans la direction de circonférence de hanche, ainsi qu'une section d'entrejambe (8), qui s'étend, dans la direction longitudinale (2), entre la section abdominale (4) et la section dorsale (6) et qui est fixée de manière indétachable à la section abdominale (4) dans une zone de chevauchement avant (14) et à la section dorsale (6) dans une zone de chevauchement arrière (16), la section d'entrejambe (8) présentant un corps absorbant (10) et un matériau de feuille arrière imperméable aux liquides (12), et la section d'entrejambe (8), la section abdominale (4) et la section dorsale (6) délimitant des ouvertures de jambe (18) de l'article d'incontinence (1), une couche de non-tissé (20), s'étendant dans la direction longitudinale (2) le long de la distance D et jusque dans la zone de chevauchement avant et la zone de chevauchement arrière (14, 16), étant prévue sur un côté supérieur (13), détourné du corps, du matériau de feuille arrière (12), la couche de non-tissé (20) se terminant, dans la direction longitudinale (2), avant une extrémité avant (44) du matériau de feuille arrière (12) et/ou avant une extrémité arrière (46) du matériau de feuille arrière (12), **caractérisé en ce que** la couche de non-tissé (20) est reliée au matériau de feuille arrière (12) au moyen d'une première et d'une deuxième bande longitudinale de colle marginale (34a, 34b), s'étendant dans la direction longitudinale (2), et au moyen d'une pluralité de premières bandes longitudinales de colle (32), situées, par rapport aux bandes longitudinales de colle marginales (34a, 34b), vers l'intérieur entre les bandes longitudinales de colle marginales (34a, 34b) dans la direction transversale (3) et s'étendant dans la direction longitudinale (2), parallèles les unes aux autres et espacées les unes des autres par des bandes longitudinales exemptes de colle (30), les bandes longitudinales de colle marginales (34a, 34b) présentant, dans la direction transversale (3), une largeur B14, les premières bandes longitudinales de colle (32) présentant une largeur B12, et les bandes longitudinales exemptes de colle présentant une largeur B10.

2. Article absorbant pour l'incontinence (1) selon la revendication 1, **caractérisé en ce que**, à l'extérieur d'un premier et d'un deuxième bord longitudinal (11a, 11b) du corps absorbant (10), le matériau de feuille arrière (12) et la couche de non-tissé (20) présentent un premier et un deuxième débord (17a, 17b) s'étendant dans la direction transversale (3), et dans lequel la première et la deuxième bande longitudinale de colle marginale (34a, 34b) sont disposées à l'intérieur du premier et du deuxième débord (17a, 17b) respectif et à une distance du bord longitudinal (11a, 11b) respectif du corps absorbant (10).

3. Article absorbant pour l'incontinence (1) selon la revendication 1 ou 2, **caractérisé en ce que** les zones marginales (9), situées des deux côtés et délimitant les ouvertures de jambe (18), de la section d'entrejambe (8) s'étendent, dans la direction longitudinale (2), exclusivement de manière rectiligne ou, au moins le long d'une partie des zones marginales (9), situées des deux côtés, de la section d'entrejambe (8), selon un contour incurvé, les bandes longitudinales de colle marginales (34a, 34b), dans le cas de zones marginales (9) exclusivement rectilignes de la section d'entrejambe (8), s'étendant de manière continue au moins sur la distance D et, en outre, en particulier certaines des premières bandes longitudinales de colle (32), et plus particulièrement toutes les premières bandes longitudinales de colle (32), s'étendant de manière continue au moins sur la distance D, ou, dans le cas d'un contour incurvé des zones marginales (9), situées des deux côtés, de la section d'entrejambe (8), les bandes longitudinales de colle marginales (34a, 34b) et, en particulier, les premières bandes longitudinales de colle (32) disposées immédiatement de manière adjacente aux bandes longitudinales de colle marginales (34a, 34b), étant interrompues, dans la direction longitudinale (2), dans la zone du contour.

4. Article absorbant pour l'incontinence (1) selon l'une des revendications précédentes, **caractérisé en ce que** les bandes longitudinales de colle marginales (34a, 34b) s'étendent dans la direction longitudinale (2) de telle sorte que les bandes longitudinales de colle marginales (34a, 34b) sont présentes dans une zone de transition avant (50) de la section d'entrejambe (8), qui s'étend, dans la direction longitudinale (2), en avant et en arrière d'un bord (5), orienté vers l'entrejambe, de la section abdominale (4), et que les bandes longitudinales de colle marginales (34a, 34b) sont présentes dans une zone de transition arrière (70) de la section d'entrejambe (8), qui s'étend, dans la direction longitudinale (2), en avant et en arrière d'un bord (7), orienté vers l'entrejambe, de la section dorsale (6).

5. Article absorbant pour l'incontinence (1) selon l'une des revendications précédentes, **caractérisé en ce qu'**un rapport de la largeur B12 des premières bandes longitudinales de colle (32) à la largeur B10 des bandes longitudinales exemptes de colle (30) immédiatement adjacentes, calculé sous la forme B12/B10, est de 0,2 à 3,0, notamment de 0,5 à 2,5, plus particulièrement de 0,7 à 2,5, plus particulièrement de 0,9 à 2,3, et plus particulièrement de 1,0 à 2,0.

6. Article absorbant pour l'incontinence (1) selon l'une des revendications précédentes, **caractérisé en ce que** la largeur B12 des premières bandes longitudinales de colle (32) est d'au moins 1 mm et d'au plus 10 mm, notamment d'au plus 8 mm, plus particulièrement d'au plus 6 mm, plus particulièrement d'au plus 4 mm, et/ou **en ce que** la largeur B10 des bandes longitudinales exemptes de colle (30) est d'au moins 1 mm et d'au plus 12 mm, notamment d'au plus 10 mm, notamment d'au plus 8 mm, plus particulièrement d'au plus 6 mm, plus particulièrement d'au plus 4 mm.

7. Article absorbant pour l'incontinence (1) selon l'une des revendications précédentes, **caractérisé en ce que** la largeur B12 des premières bandes longitudinales de colle (32) est identique pour chacune d'entre elles et/ou **en ce que** la largeur B10 des bandes longitudinales exemptes de colle (30) est identique pour chacune d'entre elles.

8. Article absorbant pour l'incontinence (1) selon l'une des revendications précédentes, **caractérisé en ce que** la largeur B14 des bandes longitudinales de colle marginales (34a, 34b) est supérieure à la largeur B12 des premières bandes longitudinales de colle (32), notamment supérieure à la largeur B12 des premières bandes longitudinales de colle (32) disposées immédiatement de manière adjacente aux bandes longitudinales de colle marginales (34a, 34b), plus particulièrement supérieure à la largeur B12 de toutes les premières bandes longitudinales de colle (32).

9. Article absorbant pour l'incontinence (1) selon la revendication 8, **caractérisé en ce que** la largeur B14 des bandes longitudinales de colle marginales (34a, 34b) est au moins égale à 1,5 fois, plus particulièrement au moins égale à 2 fois, et plus particulièrement au plus égale à 20 fois, notamment au plus égale à 15 fois, notamment au plus égale à 10 fois, notamment au plus égale à 8 fois, plus particulièrement au plus égale à 5 fois, notamment au plus égale à 4 fois la largeur B12 des premières bandes longitudinales de colle (32).

10. Article absorbant pour l'incontinence (1) selon l'une des revendications précédentes, **caractérisé en ce que** la largeur B14 des bandes longitudinales de colle marginales (34a, 34b) est d'au moins 3 mm et d'au plus 20 mm, notamment d'au plus 15 mm, plus particulièrement d'au plus 12 mm, plus particulièrement d'au plus 10 mm, plus particulièrement d'au plus 8 mm, plus particulièrement d'au plus 6 mm.

11. Article absorbant pour l'incontinence (1) selon l'une des revendications précédentes, **caractérisé en ce que** la couche de non-tissé (20) présente, dans la direction transversale (3), une largeur maximale B2, et un rapport de la largeur B12 des premières bandes longitudinales de colle (32) à la largeur B2, calculé sous la forme B12/B2, est au plus de 0,10, de préférence au plus de 0,08, de manière davantage préférée au plus de 0,06, de manière davantage préférée au plus de 0,04, de manière davantage préférée au plus de 0,02, et/ou **en ce que** la couche de non-tissé (20) présente, dans la direction transversale (3), une largeur maximale B2, et un rapport de la largeur B14 des bandes longitudinales de colle marginales (34a, 34b) à la largeur B2, calculé sous la forme B14/B2, est de 0,01 à 0,15, de préférence de 0,01 à 0,10, de préférence de 0,01 à 0,08, de manière davantage préférée de 0,01 à 0,06, de manière davantage préférée de 0,01 à 0,05, de manière davantage préférée de 0,01 à 0,04, de manière davantage préférée de 0,01 à 0,03.

12. Article absorbant pour l'incontinence (1) selon l'une des revendications précédentes, **caractérisé en ce que** la couche de non-tissé (20) présente, dans la direction longitudinale (2), une longueur L1, et le matériau de feuille arrière (12) présente, dans la direction longitudinale (2), une longueur L2, et un rapport de la longueur L1 à la longueur L2, calculé sous la forme L1/L2, est d'au moins 0,5 et, de manière davantage préférée, d'au plus 0,9, de manière davantage préférée d'au plus 0,8, de manière davantage préférée d'au plus 0,7.

13. Article absorbant pour l'incontinence (1) selon l'une des revendications précédentes, **caractérisé en ce que** la couche de non-tissé (20) se termine, dans la direction longitudinale (2), avant une extrémité avant (40) du corps absorbant (10) et/ou avant une extrémité arrière (42) du corps absorbant (10).

14. Article absorbant pour l'incontinence (1) selon l'une des revendications précédentes, **caractérisé en ce que** les premières bandes longitudinales de colle (32) et/ou les bandes longitudinales de colle marginales (34a, 34b) présentent un grammage de colle d'au moins 0,5 g/m², notamment d'au moins 0,7 g/m², plus particulièrement d'au moins 1 g/m², plus particulièrement d'au moins 1,2 g/m², et notamment d'au plus 10 g/m², plus particulièrement d'au plus 8 g/m², plus particulièrement d'au plus 6 g/m², plus particulièrement d'au plus 4 g/m².

15. Article absorbant pour l'incontinence (1) selon l'une des revendications précédentes, **caractérisé en ce que** les bandes longitudinales de colle marginales (34a, 34b) présentent un grammage de colle supérieur à celui des premières bandes longitudinales de colle (32), le grammage de colle étant notamment au moins égal à 2 fois, notamment au moins égal à 3 fois, plus particulièrement au moins égal à 4 fois, plus particulièrement au moins égal à 5 fois, et plus particulièrement au plus égal à 10 fois le grammage de colle des premières bandes longitudinales de colle (32).

16. Article absorbant pour l'incontinence (1) selon l'une des revendications précédentes, **caractérisé en ce que** la couche de non-tissé (20) recouvre le matériau de feuille arrière (12) en formant une zone de chevauchement (21), la zone de chevauchement (21) présentant une surface A1, et dans lequel les premières bandes longitudinales de colle (32) et les bandes longitudinales de colle marginales (34a, 34b) présentent, ensemble, un taux de couverture, calculé comme la surface totale des bandes longitudinales de colle (32, 34a, 34b) rapportée à la surface A1 de la zone de chevauchement (21), d'au moins 10 %, plus particulièrement d'au moins 12 %, plus particulièrement d'au moins 14 %, plus particulièrement d'au moins 16 %, et plus particulièrement d'au plus 80 %, plus particulièrement d'au plus 75 %, plus particulièrement d'au plus 70 %, plus particulièrement d'au plus 65 %.

17. Article absorbant pour l'incontinence (1) selon l'une des revendications précédentes, **caractérisé en ce que** la couche de non-tissé (20) recouvre entièrement le matériau de feuille arrière (12) dans la direction transversale (3).

18. Article absorbant pour l'incontinence (1) selon l'une des revendications précédentes, **caractérisé en ce que** la section d'entrejambe (8) est reliée à la section abdominale (4) au moyen d'une pluralité de premières bandes transversales de colle (42), prévues dans la zone de chevauchement avant (14), s'étendant dans la direction transversale (3), parallèles les unes aux autres et espacées les unes des autres par des bandes transversales exemptes de colle (40), qui sont situées à l'intérieur par rapport à d'éventuelles bandes transversales de colle marginales (44a, 44c), et/ou la section d'entrejambe (8) est reliée à la section dorsale (6) au moyen d'une pluralité de premières bandes transversales de colle (42), prévues dans la zone de chevauchement arrière (16), s'étendant dans la direction transversale (3), parallèles les unes aux autres et espacées les unes des autres par des bandes transversales exemptes de colle (40), qui sont situées à l'intérieur par rapport à d'éventuelles bandes transversales de colle marginales (44b, 44d).

19. Article absorbant pour l'incontinence (1) selon la revendication 18, **caractérisé en ce que** des bandes transversales de colle marginales (44a, 44b, 44c, 44d), s'étendant dans la direction transversale (3), sont prévues dans la zone de chevauchement avant (14) et/ou dans la zone de chevauchement arrière (16), lesdites bandes présentant, dans la direction longitudinale (2), une largeur B18, la largeur B18 étant supérieure à une largeur B16 des premières bandes transversales de colle (42), et/ou **en ce qu'**une première bande transversale de colle marginale (44a, 44b), orientée vers l'entrejambe, s'étendant dans la direction transversale (3), et une deuxième bande transversale de colle marginale (44c, 44d), orientée vers l'ouverture de hanche, s'étendant dans la direction transversale (3), ainsi qu'une pluralité de premières bandes transversales de colle (42) situées à l'intérieur entre celles-ci dans la direction longitudinale (2), sont prévues dans la zone de chevauchement avant (14) et/ou dans la zone de chevauchement arrière (16), une largeur B18 des bandes transversales de colle marginales (44a, 44b, 44c, 44d) étant supérieure à une largeur B16 des premières bandes transversales de colle (42) situées à l'intérieur par rapport aux bandes transversales de colle marginales (44a, 44b, 44c, 44d), et la largeur B18 étant notamment au moins égale à 4 fois, notamment au moins égale à 5 fois, et plus particulièrement au plus égale à 8 fois, notamment au plus égale à 7 fois la largeur B16 des premières bandes transversales de colle (42) situées à l'intérieur.

20. Article absorbant pour l'incontinence (1) selon l'une des revendications 18 à 19, **caractérisé en ce que**, dans une zone marginale orientée vers l'entrejambe de la zone de chevauchement avant (14) et/ou dans une zone marginale orientée vers l'entrejambe de la zone de chevauchement arrière (16), au moins la première et la deuxième bandes longitudinales de colle marginales (34a, 34b), et plus particulièrement certaines des premières bandes longitudinales de colle (32), plus particulièrement toutes les premières bandes longitudinales de colle (32), chevauchent une première bande transversale de colle marginale (44a, 44b), orientée vers l'entrejambe.
